(19) Europäisches Patentamt
European Patent Office
Office européen des brevets

(11) Numéro de publication : 0 495 709 A1

(12) **DEMANDE DE BREVET EUROPEEN**

(21) Numéro de dépôt : **92400095.3**

(22) Date de dépôt : **15.01.92**

(51) Int. Cl.[5] : **C07D 253/06,** A61K 31/53, C07D 403/12, C07D 405/04, C07D 487/04

(30) Priorité : **15.01.91 FR 9100338**

(43) Date de publication de la demande : **22.07.92 Bulletin 92/30**

(84) Etats contractants désignés : **AT BE CH DE DK ES FR GB GR IT LI LU NL PT SE**

(71) Demandeur : **ADIR ET COMPAGNIE**
**1 rue Carle Hébert**
**F-92415 Courbevoie Cédex (FR)**

(72) Inventeur : **Wermuth, Camille Georges**
**3, rue de la Côte d'Azur**
**F-67100 Strasbourg (FR)**
Inventeur : **Bourguignon, Jean-Jacques**
**2, rue du Feldwasser**
**F-67150 Hipsheim (FR)**
Inventeur : **Morin, Isabelle**
**25, Allée Valère Lefebvre**
**F-93340 Le Raincy (FR)**
Inventeur : **Renard, Pierre**
**50, Av. de Villeneuve l'Etang**
**F-78000 Versailles (FR)**
Inventeur : **Devissaguet, Michelle**
**14, Bld. d'Inkermann**
**F-92200 Neuilly sur Seine (FR)**
Inventeur : **Renaud de la Faverie, Jean-François**
**7, rue des Erables, Rocquencourt**
**F-78150 Le Chesnay (FR)**
Inventeur : **Adam, Gérard**
**Le Clos du Mesnil, Route du Pecq**
**F-78600 Le Mesnil Le Roi (FR)**

(54) **Nouveaux composés de structure aryltriazinique leur procédé de préparation et les compositions pharmaceutiques qui les contiennent.**

(57) Nouveaux composés de structure aryltriazinique de formule générale (I) :

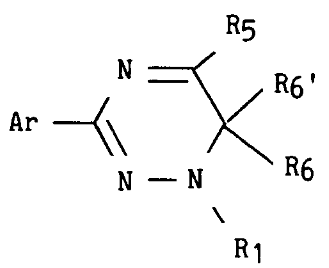

(I)

pour laquelle la signification des substituants Ar, $R_1$, $R_5$, $R_6$ et $R_6'$ est donnée dans la description,
leur procédé de préparation,
et les compositions pharmaceutiques qui les contiennent.
Les composés de l'invention sont utiles comme médicaments dans les troubles liés à un dysfonctionnement cholinergique.

EP 0 495 709 A1

Jouve, 18, rue Saint-Denis, 75001 PARIS

L'invention concerne de nouveaux composés de structure aryltriazinique, leur procédé de préparation et les compositions pharmaceutiques qui les contiennent.

On connait de la littérature des composés de structure aryltriazinique, notamment des aryltriazine-1,2,4 ones-6 mentionnées comme herbicides, et des aryltriazolo[3,4-f] [1,2,4]triazines dont les activités pharmacologiques n'ont pas été recherchées. La demanderesse a désormais découvert de nouveaux composés aryltriaziniques qui possèdent de façon très intéressante des propriétés analgésiques, et inhibitrices de l'acétylcholinestérase ainsi que des affinités importantes pour certains récepteurs centraux, et à ce titre ils peuvent être utilisés avec profit en médecine humaine. L'état de l'art le plus proche des composés de l'invention est constitué par les documents suivants : Hétérocycles, (1989), **29** (12), 2279-2285 ; US 4,362,550 ; Chem. Abstracts 105 : 172410 ; EP 169139.

Plus spécifiquement l'invention concerne des composés de structure aryltriazinique répondant à la formule générale I :

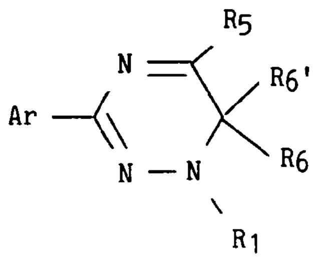

(I)

dans laquelle :

– Ar représente un radical aryle ou hétéroaryle, éventuellement substitué ,

– $R_5$ représente un atome d'hydrogène, un radical alkyle, cycloalkyle , cycloalkylalkyle, aryle, aralkyle, hétéroaryle, ou hétéroaralkyle éventuellement substitué sur le cycle aromatique ou hétéroaromatique, et

soit :

– $R_6$ et $R_6'$ représentent ensemble avec l'atome de carbone qui les porte une fonction C=O , et

– $R_1$ représente une chaîne

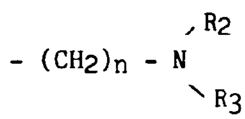

dans laquelle :

– n représente 2, 3, ou 4 ,

– $R_2$ et $R_3$, identiques ou différents, représentent un atome d'hydrogène, un radical alkyle, ou forment ensemble, avec l'atome d'azote qui les porte, un hétérocycle choisi parmi morpholine, pyrrolidine, pipéridine, perhydroazépine, et pipérazine éventuellement substituée au niveau de l'autre atome d'azote par une chaîne alkyle ou un radical phényle éventuellement substitué, soit :

– $R_1$ et $R_6$ forment ensemble une liaison, et

– $R_6'$ représente un radical de formule :

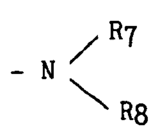

dans laquelle

. $R_7$ représente un atome d'hydrogène ou un radical alkyle,

. et $R_8$ représente un groupement de formule :

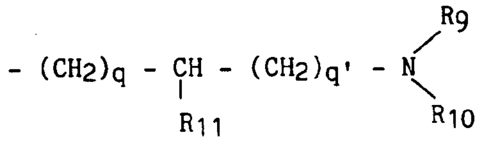

dans lequel

. q représente un nombre entier de 1 à 3,

. q′ représente 0 ou 1,

. $R_9$, $R_{10}$, et $R_{11}$ représentent chacun indépendamment l'un de l'autre un atome d'hydrogène ou un alkyle,

. ou $R_9$ et $R_{10}$ forment ensemble avec l'atome d'azote qui les porte un hétérocycle de 5 à 10 sommets, mono ou bi-cyclique, saturé ou possédant une double liaison, incluant éventuellement dans son squelette un autre hétéroatome choisi parmi azote, oxygène, et soufre, étant entendu que lorsque $R_9$ et $R_{10}$ forment ensemble un hétérocycle contenant un second atome d'azote, cet atome d'azote peut également être substitué par un radical alkyle, aryle ou hétéroaryle éventuel-lement substitué, ou par une chaîne de formule

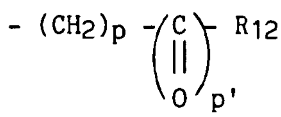

dans laquelle p représente 1, 2, ou 3, et p′ représente 0 ou 1, $R_{12}$ représente un radical aryle ou hétéroaryle éventuellement substitué,

. ou $R_9$ et $R_{11}$ forment ensemble avec les atomes d'azote et de carbone qui les portent un hétérocycle de 5 à 7 sommets incluant éventuellement un autre hétéroatome dans son squelette un autre hétéroatome choisi parmi azote, oxygène, et soufre,

. ou $R_7$ et $R_8$ forment ensemble, avec l'atome d'azote qui les porte, une pipérazine substituée sur l'autre atome d'azote par un radical alkyle, par un radical phényle éventuellement substitué, ou par une chaîne de formule

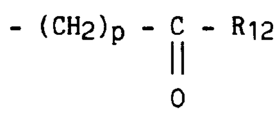

dans laquelle p et $R_{12}$ sont tels que définis précédemment, soit :

$R_1$, $R_6$, et $R_6'$ forment avec le noyau triazinique qui les porte un système [1,2,4-triazolo][3,4-f][1,2,4]-triazine, le noyau triazolique étant substitué en position 3 par un groupement $R_{13}$ choisi parmi un radical alkyle, aryle, hétéroaryle, aralkyle, ou hétéroaralkyle éventuellement substitués sur le cycle aromatique ou hétéroaromatique,

leurs éventuels isomères optiques, isolés ou sous forme de mélange, ainsi que, le cas échéant, leurs sels d'addition à un acide pharmaceutiquement acceptable,

étant entendu que :

– le terme "substitué" signifie que les groupements qu'il affecte peuvent être substitués par un ou plusieurs radicaux, identiques ou différents, choisis parmi halogène, hydroxy, nitro, amino, trifluorométhyle, alkyl, et alkoxy, et/ou peuvent porter sur 2 atomes de carbone adjacents un groupement $-O-(CH_2)_r-O-$ dans lequel r représente un nombre entier de 1 à 3,

– sauf précision contraire, les termes "alkyle" et "alkoxy" signifient des groupements contenant de 1 à 6 atomes de carbone en chaîne droite ou ramifiée, et le terme "cycloalkyle" représente un groupement cyclique saturé comprenant de 3 à 7 atomes de carbone,

– le terme "aryle" représente un phényle ou un naphtyle, et le terme "hétéroaryle" représente un groupement aromatique, mono ou bicyclique, comprenant de 5 à 10 sommets, et incluant dans son squelette carboné de 1 à 3 hétéroatomes choisis parmi azote, oxygène, et soufre.

L'invention s'étend également au procédé de préparation des dérivés de formule I, caractérisé en ce que l'on condense en milieu alcalin un α-aminoacide de formule II

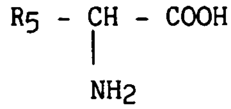

(II)

dans laquelle $R_5$ a la même signification que dans la formule I, avec un halogénure d'acide de formule Ar - COX, dans laquelle Ar a la même signification que dans la formule I et X représente un atome d'halogène, pour obtenir un composé de formule III

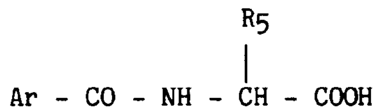

(III)

dans laquelle Ar et $R_5$ ont les significations ci-dessus définies, qui est ensuite :

a) **soit** estérifié en milieu acide par un alcool inférieur de formule R'-OH dans laquelle R' représente un alkyle inférieur, pour conduire à un composé de formule IVa

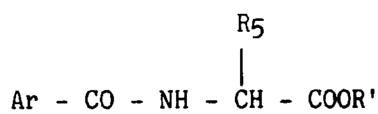

(IVa)

dans laquelle Ar, $R_5$, et R' ont les significations ci-dessus définies, qui est

– ou bien soumis en milieu anhydre à l'action d'un réactif de thionation tel que le pentasulfure de phosphore ou le réactif de Lawesson suivie d'une acidification ou d'une extraction acido-basique pour obtenir un composé de formule Va

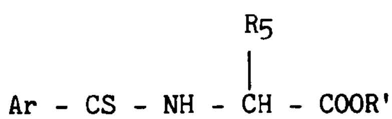

(Va)

dans laquelle Ar, $R_5$, et R' ont les significations ci-dessus définies,

– ou bien soumis à l'action d'un tétrafluoroborate de trialkyloxonium , dans un solvant anhydre, pour obtenir un composé de formule Vb

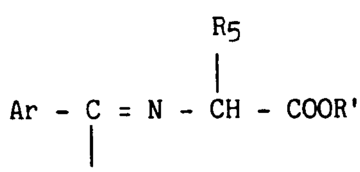

(Vb)

dans laquelle Ar, $R_5$ et R' ont les significations ci-dessus définies, et R" représente un groupement alkyle, les composés Va ou Vb étant ensuite cyclisés à chaud dans un solvant aprotique par action de l'hydrazine en un composé de formule VI

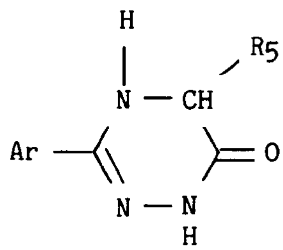

(VI)

dans laquelle Ar et $R_5$ ont les significations ci dessus définies, qui est ensuite deshydrogéné, soit par dehydrohalogénation, soit par action d'un réactif oxydant comme par exemple le permanganate de potassium, le dioxyde de manganèse, le métanitrobenzosulfonate de sodium, ou le perchlorate de sodium, en un composé de formule VII

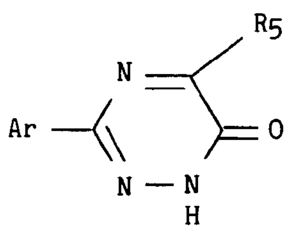

(VII)

dans laquelle Ar et $R_5$ ont la même signification que précédemment, qui est ensuite :

* soit traité successivement par un alcoolate de métal alcalin puis par un halogénure d'aminoalkyle de formule VIII

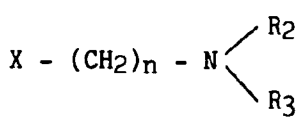

(VIII)

dans laquelle n, $R_2$ et $R_3$ ont la même signification que dans la formule I et X représente un atome d'halogène, pour obtenir un composé de formule Ia

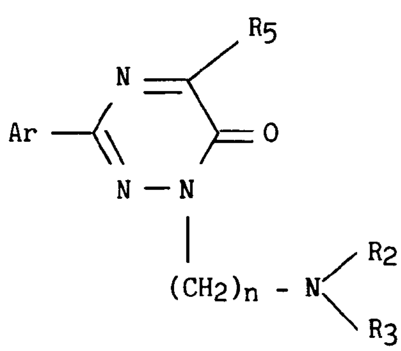

(Ia)

cas particulier des composés de formule I,

* soit soumis à l'action d'un composé polyhalogéné du phosphore, pour obtenir un composé de formule IX

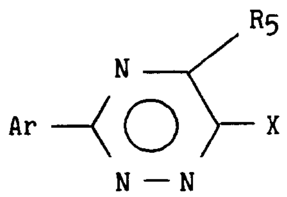

(IX)

dans laquelle Ar, $R_5$, et X ont les significations ci-dessus définies, qui est

– ou bien condensé avec une amine de formule X

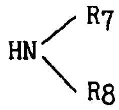

(X)

dans laquelle $R_7$ et $R_8$ ont la même signification que dans la formule I, pour obtenir un composé de formule Ib

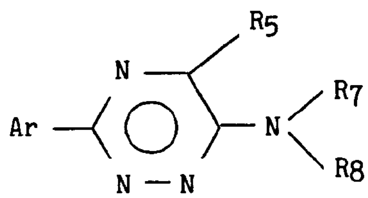

(Ib)

cas particulier des composés de formule I,

– ou bien condensé avec un hydrazide de formule XI

$$R_{13} - CO - NH - NH_2 \qquad (XI)$$

dans laquelle $R_{13}$ a la même signification dans la formule I, pour obtenir un composé de formule Ic

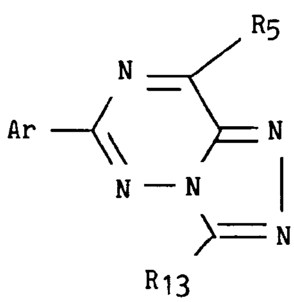

(Ic)

cas particulier des composés de formule I,

les composés Ia, Ib,et Ic qui forment l'ensemble des composés de formule I pouvant être si l'on désire salifiés par addition d'un acide pharmaceutiquement acceptable, et le cas échéant séparés en leurs isomères optiques,

b) **soit,** éventuellement, lorsque $R_5$ représente un atome d'hydrogène, condensé dans l'anhydride acétique en présence d'un acétate de métal alcalin, avec un aldéhyde aromatique de formule $Ar_1$ - CHO, dans laquelle $Ar_1$ représente un radical aryle ou hétéroaryle éventuellement substitué, pour obtenir un composé de formule Vc

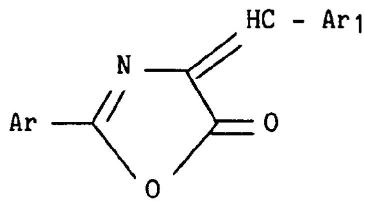

(Vc)

dans laquelle Ar et $Ar_1$ ont les significations ci dessus définies,
qui est soumis à l'action de l'hydrazine pour obtenir un composé de formule Vd

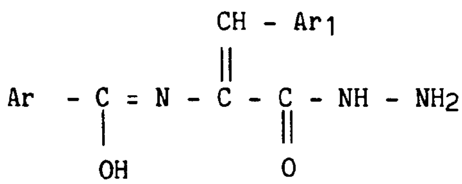

(Vd)

dans laquelle Ar et $Ar_1$ possèdent les significations ci dessus définies, qui chauffé en milieu alcalin conduit au composé de formule VIIa

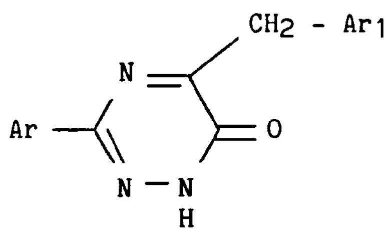

(VIIa)

cas particulier des composés de formule VII pour lesquels $R_5$ représente un radical arylméthyle ou hétéroaryl méthyle éventuellement substitué,
qui est soumis aux même opérations que les composés de formule générale VII pour obtenir respectivement les composés de formule Ia', Ib', et Ic',

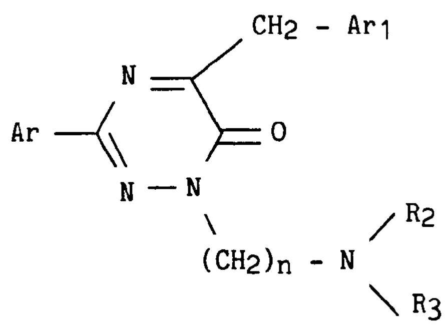

(Ia')

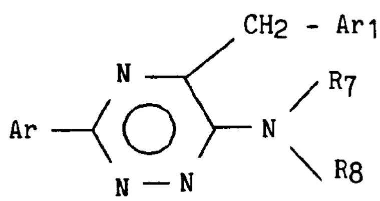

(Ib')

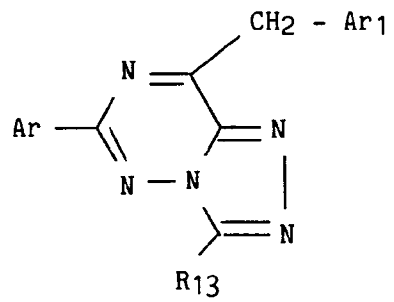

(Ic')

cas particuliers des composés de formule Ia, Ib, et Ic, qui sont, si l'on désire, salifiés par addition d'un acide pharmaceutiquement acceptable et, le cas échéant, séparés en leurs isomères optiques.

Les composés de formule I possèdent des propriétés pharmacologiques intéressantes.

Les essais de liaison in vitro ont montré que les composés ont une bonne affinité pour les récepteurs $A_1$, $A_2$, $M_1$ et sigma. Par ailleurs, ils possèdent une puissante activité inhibitrice de l'acétylcholinestérase.

In vivo, les tests pratiqués montrent qu'il s'agit de substances atoxiques aux doses testées, possédant également une activité analgésique.

Les composés de l'invention trouvent donc leur application dans le traitement de la douleur, des troubles cognitifs liés ou non à la sénescence, de la dépression, de l'anxiété et des psychoses, des troubles du sommeil, de l'épilepsie, de l'hypertension artérielle, de l'insuffisance cardiaque ou de l'arythmie cardiaque, de l'ischémie cardiaque ou cérébrale, de l'hypercoagulabilité sanguine et des thromboses, de l'asthme, de l'insuffisance rénale, et en général des désordres liés à un dysfonctionnement des récepteurs A1, A2, ou sigma, ou du système cholinergique.

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, les solutés injectables, etc...

La posologie varie selon l'âge et le poids du patient, la nature et la sévérité de l'affection, ainsi que la voie d'administration. Celle ci peut être orale, nasale, rectale, intramusculaire, ou parentérale.

D'une manière générale, la posologie unitaire s'échelonne entre 1 et 500 mg pour un traitement en 1 à 3 prises par 24 heures.

Les exemples suivants illustrent l'invention, mais ne la limitent en aucune façon.

Les produits de départ sont décrits dans la littérature ou peuvent être préparés de façon identique.

**EXEMPLE 1 : MORPHOLINOETHYL-1 ISOPROPYL-5 PHENYL-3 TRIAZINE-1,2,4 ONE-6**

STADE A : N-BENZOYLVALINE

Dissoudre 0,1 mole (11,7 g) de valine dans 50 ml d'eau, ajouter 0,25 mole d'hydroxyde de sodium en solution aqueuse, chauffer à 30° C puis ajouter goutte à goutte 0,15 mole (17,5 ml) de chlorure de benzoyle. Laver la solution aqueuse à l'éther, acidifier et essorer les cristaux de N-benzoylvaline

Point de fusion : 125° C

Rendement : 95 %

STADE B : N-BENZOYLVALINATE D'ETHYLE

Dissoudre 0,045 mole (10 g) de N-benzoylvaline obtenue au stade précédent dans 100 ml d'éthanol, ajouter 5 ml d'acide sulfurique concentré, puis chauffer à reflux pendant 24 heures. Evaporer à sec, reprendre par de l'éther, laver à l'eau, puis par une solution de bicarbonate de potassium à 10 %, sécher, filtrer et évaporer à sec.

On obtient le N-benzoyl valinate d'éthyle

Rendement : 71 %

Point de fusion : 74° C

STADE C : N-THIOBENZOYL VALINATE D'ETHYLE

Chauffer à reflux pendant 6 heures une solution de 0,025 mole (6,3 g) de N-benzoyl valinate d'éthyle obtenu au stade B, et de 5,6 g de pentasulfure de phosphore dans 120 ml de pyridine, évaporer à sec. Reprendre le résidu par de l'eau et de l'acétate d'éthyle. Laver la phase organique avec une solution d'acide chlorhydrique 2N, la sécher, filtrer et évaporer à sec. Le N-thiobenzoyl valinate d'éthyle obtenu est purifié par chromatographie sur colonne de silice (éluant : acétate d'éthyle/hexane : 25/75).

Rendement : 45 % (huile jaune)

STADE D : DIHYDRO-4,5 PHENYL-3 ISOPROPYL-5 TRIAZINE-1,2,4 ONE-6

Dissoudre 0,01 mole (2,85 g) de N-thiobenzoyl valinate d'éthyle obtenu au stade C dans 25 ml de xylène. Ajouter 0,02 mole d'hydrate d'hydrazine. Porter à reflux pendant 12 heures. Essorer le précipité formé et le laver à l'acétone.

On obtient la dihydro-4,5 phényl-3 isopropyl-5 triazine-1,2,4 one-6

Rendement : 61 %

Point de fusion : 164° C

STADE E : PHENYL-3 ISOPROPYL-5 TRIAZINE-1,2,4 ONE-6

Dissoudre 0,01 mole (2,15 g) du composé obtenu au stade précedent, dans 400 ml d'acétone. Ajouter 8 grammes de permanganate de potassium en solution dans 400 ml d'eau, porter à reflux pendant 4 heures. Filtrer, évaporer à sec et recristalliser la phényl-3 isopropyl-5 triazine-1,2,4 one-6 dans le méthanol.

Rendement : 50 %

Point de fusion : 188° C

STADE F : MORPHOLINOETHYL-1 ISOPROPYL-5 PHENYL-3 TRIAZINE-1,2,4 ONE-6,(OXALATE ACIDE)

A une solution dans l'éthanol de 4,6 mmoles (1 gramme) du composé obtenu au stade précedent, ajouter 5 mmoles d'éthylate de sodium, puis une solution dans l'éthanol de 4,6 mmoles de chlorhydrate de N-(chloro-2 éthyl) morpholine et de 5 mmoles d'éthylate de sodium. Chauffer à reflux pendant 6 heures, filtrer le chlorure de sodium formé. Evaporer à sec le filtrat, reprendre par de l'eau et extraire par de l'acétate d'éthyle. Concentrer à sec, dissoudre le résidu dans de l'isopropanol à chaud. Ajouter un équivalent d'acide oxalique. L'oxalate acide de la morpholinoéthyl-1 isopropyl-5 phényl-3 triazine-1,2,4 one-6 précipite.

Rendement : 68 %

Point de fusion : 210° C

Analyse centésimale :

| | C % | H % | N % |
|---|---|---|---|
| calculé | 57,40 | 6,26 | 13,39 |
| trouvé | 57,46 | 6,33 | 13,24 |

**EXEMPLE 2 : MORPHOLINOETHYL-1 ISOPROPYL-5 (CHLORO-2 PHENYL)-3 TRIAZINE-1,2,4 ONE-6**

En remplaçant dans l'exemple 1, stade A, le chlorure de benzoyle par le chlorure de l'acide chloro-2 benzoïque, on obtient successivement et de la même façon :

STADE A : la N-(CHLORO-2 BENZOYL) VALINE

Rendement : 78 %

Point de fusion : 89° C

STADE B : le N-(CHLORO-2 BENZOYL) VALINATE D'ETHYLE

Rendement : 76 % (huile)

STADE C : le N-(CHLORO-2 THIOBENZOYL) VALINATE D'ETHYLE

Rendement : 50 % (huile jaune)

STADE D : la DIHYDRO-4,5 (CHLORO-2 PHENYL)-3 ISOPROPYL-5 TRIAZINE 1,2,4 ONE-6

Rendement : 66 %
Point de fusion : 98° C

STADE E : la (CHLORO-2 PHENYL)-3 ISOPROPYL-5 TRIAZINE-1,2,4 ONE-6

Rendement : 56 %
Point de fusion : 118,5° C

STADE F : la MORPHOLINOETHYL-1 (CHLORO-2 PHENYL)-3 ISOPROPYL-5 TRIAZINE-1,2,4 ONE-6 sous forme d'oxalate acide

Rendement : 50 %
Point de fusion : 188° C
Analyse centésimale :

| | C % | H % | N % |
|---|---|---|---|
| calculé | 53,03 | 5,56 | 12,37 |
| trouvé | 52,97 | 5,64 | 12,27 |

**EXEMPLE 3 : (MORPHOLINO-3 PROPYL)-1 (CHLORO-2 PHENYL)-3 ISOPROPYL-5 TRIAZINE-1,2,4 ONE-6**

En remplaçant dans l'exemple 2, stade F, la chloro-2 éthyl morpholine par la N-(chloro-3 propyl)morpholine, on obtient le produit de l'exemple sous forme d'oxalate.
Point de fusion : 172° C

**EXEMPLE 4 : PYRROLIDINOETHYL-1 (CHLORO-2 PHENYL)-3 ISOPROPYL-5 TRIAZINE-1,2,4 ONE-6**

En remplaçant dans l'exemple précédent, la N-(chloro-3 propyl) morpholine par la N-(chloro-2 éthyl) pyrrolidine, on obtient le composé de l'exemple sous forme d'oxalate acide
Point de fusion : 179° C

**EXEMPLES 5 A 8**

En procédant comme dans les exemples précédents, mais en utilisant une halogénoalkylamine appropriée, on obtient de la même façon les composés suivants sous forme d'oxalate.

**EXEMPLE 5 : (MORPHOLINO-4 BUTYL)-1 (CHLORO-2 PHENYL)-3 ISOPROPYL-5 TRIAZINE-1,2,4 ONE-6**

**EXEMPLE 6 : [(METHYL-4 PIPERAZINYL-1) ETHYL]-1 (CHLORO-2 PHENYL)-3 ISOPROPYL-5 TRIAZINE-1,2,4 0NE-6**

**EXEMPLE 7 : [[(TRIFLUOROMETHYL-3 PHENYL)-4 PIPERAZINYL-1] ETHYL]- (CHLORO-2 PHENYL)-3 ISOPROPYL-5 TRIAZINE-1,2,4 ONE-6**

**EXEMPLE 8 : DIETHYLAMINOETHYL-1 (CHLORO-2 PHENYL)-3 ISOPROPYL-5 TRIAZINE-1,2,4 ONE-6**

**EXEMPLE 9 : MORPHOLINOETHYL-1 (METHOXY-3 PHENYL)-3 ISOPROPYL-5 TRIAZINE-1,2,4 ONE-6**

En remplaçant dans l'exemple 1, stade A, le chlorure de benzoyle par le chlorure de l'acide méthoxy-3 benzoïque et en procédant de la même façon, on obtient le produit de l'exemple, sous forme d'oxalate acide.
Point de fusion : 117° C

**EXEMPLE 10 : MORPHOLINOETHYL-1 (CHLORO-4 PHENYL)-3 ISOPROPYL-5 TRIAZINE-1,2,4 ONE-6**

En remplaçant dans l'exemple précédent le chlorure de l'acide méthoxy-3 benzoique par le chlorure de l'acide chloro-4 benzoique, on obtient le composé de l'exemple, sous forme d'oxalate acide .
Point de fusion : 213° C

**EXEMPLE 11 : MORPHOLINOETHYL-1 (CHLORO-3 PHENYL)-3 ISOPROPYL-5 TRIAZINE-1,2,4 ONE-6**

En remplaçant dans l'exemple précedent le chlorure de l'acide chloro-4 benzoïque par le chlorure de l'acide chloro-3 benzoique, on obtient le composé de l'exemple, sous forme d'oxalate acide .
Point de fusion : 211° C

**EXEMPLE 12 : MORPHOLINOETHYL-1 PHENYL-3 METHYL-5 TRIAZINE-1,2,4 ONE-6**

En remplaçant dans l'exemple 1, stade A la valine par l'alanine, on obtient successivement
la N-BENZOYL ALANINE (Point de fusion : 98,5° C)
la N-BENZOYL ALANINATE D'ETHYLE (Point de fusion : 70° C)
la N-THIOBENZOYL ALANINATE D'ETHYLE (Point de fusion : 119° C)
la DIHYDRO-4,5 PHENYL-3 METHYL-5 TRIAZINE-1,2,4 ONE-6 (Point de fusion : 177° C)
la PHENYL-3 METHYL-5 TRIAZINE-1,2,4 ONE-6 (Point de fusion : 230° C)
la MORPHOLINOETHYL-1 PHENYL-3 METHYL-5 TRIAZINE-1,2,4 ONE-6 (Point de fusion : 75 - 76° C sous forme de base)

**EXEMPLE 13 : MORPHOLINOETHYL-1 (CHLORO-3 PHENYL)-3 METHYL-5 TRIAZINE-1,2,4 ONE-6**

En remplaçant dans l'exemple précedent le chlorure de benzoyle par le chlorure de chloro-3 benzoyle, on obtient de la même façon le produit de l'exemple sous forme d'oxalate acide.
Point de fusion : 209° C

**EXEMPLE 14 : MORPHOLINOETHYL-1 (CHLORO-4 PHENYL)-3 METHYL-5 TRIAZINE-1,2,4 ONE-6**

En remplaçant dans l'exemple 1, le chlorure de benzoyle par le chlorure de chloro-4 benzoyle, et la valine par l'alanine, on obtient successivement :

STADE A : N-(CHLORO-4 BENZOYL) ALANINE

Point de fusion : 178° C

STADE B : N-(CHLORO-4 BENZOYL) ALANINATE D'ETHYLE

Point de fusion : 88° C

STADE C : N-[(CHLORO-4 BENZOYL) ETHOXY METHYLIDENE] ALANINATE D'ETHYLE

A une solution dans le dichlorométhane de 0,05 mole (13 g) de N-(chloro-4 benzoyl) alaninate d'éthyle obtenu au stade précédent, ajouter une solution de 0,1 mole (18,9 g) de tétrafluoroborate de triéthyloxonium dans le dichlorométhane. Agiter pendant 12 heures sous atmosphère inerte. Laver la solution organique par une solution de carbonate de potassium. Sécher et évaporer la phase organique.
On obtient une huile jaune qui est utilisée telle qu'elle et immédiatement dans le stade suivant.

STADE D : DIHYDRO-4,5 (CHLORO-4 PHENYL)-3 METHYL-5 TRIAZINE-1,2,4 ONE-6

A une solution éthanolique de 0,01 mole du produit obtenu au stade précédent, ajouter 0,012 mole d'hydrate d'hydrazine. Chauffer à reflux pendant 6 heures, évaporer à sec. Reprendre par de l'eau, essorer, sécher et recristalliser dans le méthanol.
Point de fusion : 144° C

STADES E et F :

En opérant comme dans l'exemple 1, stades E et F, à partir du produit obtenu au stade précédent, on obtient de la même façon
– la (CHLORO-4 PHENYL)-3 METHYL-5 TRIAZINE-1,2,4 ONE-6
– la MORPHOLINOETHYL-1 (CHLORO-4 PHENYL)-3 METHYL-5 TRIAZINE-1,2,4 ONE-6
point de fusion : 109,5° C (base)

**EXEMPLE 15 : (CHLORO-2 PHENYL)-3 ISOBUTYL-5 MORPHOLINOETHYL-1 TRIAZINE-1,2,4 ONE-6**

En remplaçant dans l'exemple précédent, stade A, le chlorure de chloro-4 benzoyle par le chlorure de chloro-2 benzoyle, et l'alanine par la leucine, on obtient successivement :
– la N-(CHLORO-2 BENZOYL) LEUCINE
Point de fusion : 73° C
– la N-(CHLORO-2 BENZOYL) LEUCINATE D'ETHYLE (huile)
– la N-[(CHLORO-2 PHENYL) ETHOXY METHYLIDENE] LEUCINATE D'ETHYLE
– la DIHYDRO-4,5 (CHLORO-2 PHENYL)-3 ISOBUTYL-5 TRIAZINE-1,2,4 ONE-6
Point de fusion : 113° C
– la (CHLORO-2 PHENYL)-3 ISOBUTYL-5 TRIAZINE-1,2,4 ONE-6 (huile)
– la MORPHOLINOETHYL-1 (CHLORO-2 PHENYL)-3 ISOBUTYL-5 TRIAZINE-1,2,4 ONE-6 sous forme d'oxalate acide
Point de fusion : 175° C

**EXEMPLES 16 A 21**

En remplaçant dans l'exemple 14, stade A l'alanine par un aminoacide approprié, on obtient de la même façon

**EXEMPLE 16 : MORPHOLINOETHYL-1 (CHLORO-2 PHENYL)-3 ETHYL-5 TRIAZINE-1,2,4 ONE-6**

**EXEMPLE 17 : MORPHOLINOETHYL-1 (CHLORO-2 PHENYL)-3 ALLYL-5 TRIAZINE-1,2,4 ONE-6**

**EXEMPLE 18 : MORPHOLINOETHYL-1 (CHLORO-2 PHENYL)-3 (THIENYL-2)-5 TRIAZINE-1,2,4 ONE-6**

**EXEMPLE 19 : MORPHOLINOETHYL-1 (CHLORO-2 PHENYL)-3 HEXYL-5 TRIAZINE-1,2,4 ONE-6**

**EXEMPLE 20 : MORPHOLINOETHYL-1 (CHLORO-2 PHENYL)-3 (PHENYL-2 ETHYL)-5 TRIAZINE-1,2,4 ONE-6**

**EXEMPLE 21 : MORPHOLINOETHYL-1 (CHLORO-2 PHENYL)-3 TRIAZINE-1,2,4 ONE-6**

**EXEMPLES 22 A 27**

En remplaçant dans l'exemple 1 stade A, le chlorure de benzoyle par le chlorure d'acide approprié, on obtient de la même façon :

**EXEMPLE 22 : MORPHOLINOETHYL-1 (NAPHTYL-1)-3 ISOPROPYL-5 TRIAZINE-1,2,4 ONE-6**

**EXEMPLE 23 : MORPHOLINOETHYL-1 (PYRIDYL-3)-3 ISOPROPYL-5 TRIAZINE-1,2,4 ONE-6**

**EXEMPLE 24 : MORPHOLINOETHYL-1 (THIENYL-2)-3 ISOPROPYL-5 TRIAZINE-1,2,4 ONE-6**

**EXEMPLE 25 : MORPHOLINOETHYL-1 (FURYL-2)-3 ISOPROPYL-5 TRIAZINE-1,2,4 ONE-6**

**EXEMPLE 26 : MORPHOLINOETHYL-1 (METHYLENEDIOXY-3,4 PHENYL)-3 ISOPROPYL-5 TRIAZINE-1,2,4 ONE-6**

**EXEMPLE 27 : MORPHOLINOETHYL-1 (ETHYLENEDIOXY-3,4 PHENYL)-3 ISOPROPYL-5 TRIAZINE-1,2,4 ONE-6**

**EXEMPLE 28 : MORPHOLINOETHYL-1 PHENYL-3 BENZYL-5 TRIAZINE-1,2,4 ONE-6**

STADE A : PHENYL-2 BENZYLIDENE-4 OXAZOLINONE-5

Mettre en suspension dans 100 ml d'anhydride acétique 0,46 mole (38 g) d'acétate de sodium et 0,26 mole (26,4 ml) de benzaldehyde, chauffer à 65°C, ajouter 0,32 mole (59 g) d'acide hippurique puis chauffer à 90°C pendant 30 minutes. Ajouter en 10 minutes 200 ml d'eau chaude (80°C). Maintenir l'agitation pendant 10 minutes, filtrer, laver le précipité obtenu avec de l'eau chaude. Sécher .

On obtient 0,18 mole (45 g) de phényl-2 benzylidène-4 oxazolinone-5.

Point de fusion : 158° C

STADE B : HYDRAZIDE DE L'ACIDE PHENYLCARBOXAMIDO-2 PHENYL-3 ACRYLIQUE.

Mettre en suspension 0,08 mole (20 g) de phényl-2 benzylidène-4 oxazolinone-5 dans 125 $cm^3$ de méthanol. Ajouter 0,09 mole (5,8 g) d'hydrate d'hydrazine. Agiter pendant 30 minutes, filtrer le précipité formé, le rincer à l'éther.

On obtient 0,048 mole (13,5 g) de l'hydrazide de l'acide phénylcarboxamido-2 phényl-3 acrylique.

Point de fusion : 136° C

STADE C : PHENYL-3 BENZYL-5 TRIAZINE-1,2,4 ONE-6

Porter à reflux pendant 10 minutes 0,08 mole (22,5 g) du composé obtenu au stade précedent dissous dans 150 ml d'une solution molaire d'hydroxyde de sodium. Filtrer à chaud. Acidifier le précipité formé et le recristalliser dans l'éthanol. On recueille 0,048 mole (12,6 g) de phényl-3 benzyl-5 triazine-1,2,4 one-6.

Point de fusion : 183° C

STADE D : MORPHOLINOETHYL-1 PHENYL-3 BENZYL-5 TRIAZINE-1,2,4 ONE-6

En opérant comme dans l'exemple 1, stade F mais en remplaçant la phényl-3 isopropyl-5 triazine-1,2,4 one-6 par la phényl-3 benzyl-5 triazine- 1,2,4 one-6 obtenue au stade précédent, on obtient le composé de l'exemple.

Point de fusion : 92° C (base)

Le composé de l'exemple peut être obtenu également en utilisant le procédé de l'exemple 1, mais en utilisant la phénylalanine à la place de la valine.

**EXEMPLE 29 : MORPHOLINOETHYL-1 (CHLORO-3 PHENYL)-3 BENZYL-5 TRIAZINE-1,2,4 ONE-6**

En remplaçant dans l'exemple 28, stade A l'acide hippurique par la N-(chloro-3 benzoyl) glycine on obtient successivement :

STADE A : la (CHLORO-3 PHENYL)-2 BENZYLIDENE-4 OXAZOLINONE-5

Point de fusion : 134° C

STADE B : 1' HYDRAZIDE DE L'ACIDE [(CHLORO-3 PHENYL) CARBOXAMIDO]-2 PHENYL-3 ACRYLIQUE

STADE C : la (CHLORO-3 PHENYL)-3 BENZYL-5 TRIAZINE-1,2,4 ONE-6

STADE D : la MORPHOLINO ETHYL-1 (CHLORO-3 PHENYL)-3 BENZYL-5 TRIAZINE-1,2,4 ONE-6

Point de fusion : 183° C (base libre hémihydrate)

**EXEMPLES 30 A 35**

En procédant comme dans l'exemple 29 et en utilisant une N-aroyl glycine convenablement substituée, on obtient les composés des exemples suivants :

**EXEMPLE 30 : MORPHOLINOETHYL-1 (CHLORO-4 PHENYL)-3 BENZYL-5 TRIAZINE-1,2,4 ONE-6**

Point de fusion : 189° C (oxalate acide)

**EXEMPLE 31 : MORPHOLINOETHYL-1 (METHYLENEDIOXY-3,4 PHENYL)-3 BENZYL-5 TRIAZINE-1,2,4 ONE-6**

Point de fusion : 169° C (oxalate acide)

**EXEMPLE 32 : MORPHOLINOETHYL-1 (METHOXY-4 PHENYL)-3 BENZYL-5 TRIAZINE-1,2,4 ONE-6**

Point de fusion : 107° C (base)

**EXEMPLE 33 : MORPHOLINOETHYL-1 (METHYL-4 PHENYL)-3 BENZYL-5 TRIAZINE-1,2,4 ONE-6**

Point de fusion : 200° C (oxalate acide)

**EXEMPLE 34 : MORPHOLINOETHYL-1 (NAPHTYL-1)-3 BENZYL-5 TRIAZINE-1,2,4 ONE-6**

**EXEMPLE 35 : MORPHOLINOETHYL-1 (PYRIMIDINYL-2)-3 BENZYL-5 TRIAZINE-1,2,4 ONE-6**

**EXEMPLE 36 : MORPHOLINOETHYL-1 PHENYL-3 (CHLORO-3 BENZYL)-5 TRIAZINE-1,2,4 ONE-6**

En remplaçant dans l'exemple 28, Stade A le benzaldéhyde par le chloro-3 benzaldéhyde, on obtient de la même façon :

STADE A : la PHENYL-2 (CHLORO-3 BENZYLIDENE)-4 OXAZOLINONE-5

Point de fusion : 166° C

STADE B : l'HYDRAZIDE DE L'ACIDE PHENYLCARBOXAMIDO-2 (CHLORO-3 PHENYL)-3 ACRYLIQUE

Point de fusion : 147° C

STADE C : la PHENYL-3 (CHLORO-3 BENZYL)-5 TRIAZINE-1,2,4 ONE-6

Point de fusion : 150° C

STADE D : la MORPHOLINOETHYL-1 PHENYL-3 (CHLORO-3 BENZYL)-5 TRIAZINE-1,2,4 ONE-6

Point de fusion : 187° C (oxalate acide)

**EXEMPLES 37 A 47**

En procédant comme dans l'exemple 36 mais en utilisant un aldéhyde convenablement choisi, on obtient de la même façon les composés des exemples suivants .

**EXEMPLE 37 : MORPHOLINOETHYL-1 PHENYL-3 (CHLORO-2 BENZYL)-5 TRIAZINE-1,2,4 ONE-6**

Point de fusion : 197° C (oxalate acide)

**EXEMPLE 38 : MORPHOLINOETHYL-1 PHENYL-3 (CHLORO-4 BENZYL)-5 TRIAZINE-1,2,4 ONE-6**

Point de fusion : 216° C (oxalate acide)

**EXEMPLE 39 : MORPHOLINOETHYL-1 PHENYL-3 (METHOXY-4 BENZYL)-5 TRIAZINE-1,2,4 ONE-6**

Point de fusion : 200° C (oxalate acide)

**EXEMPLE 40 : MORPHOLINOETHYL-1 PHENYL-3 (BROMO-3 BENZYL)-5 TRIAZINE1,2,4 ONE-6**

Point de fusion : 187° C (oxalate acide)

**EXEMPLE 41 : MORPHOLINOETHYL- 1 PHENYL-3 (METHYLENEDIOXY-3,4 PHENYL)-5 TRIAZINE-1,2,4 ONE-6**

Point de fusion : 151° C (base)

**EXEMPLE 42 : MORPHOLINOETHYL-1 PHENYL-3 [(INDOLYL-3) METHYL]-5 TRIAZINE-1,2,4 ONE**

**EXEMPLE 43 : MORPHOLINOETHYL-1 PHENYL-3 [(PYRIDINYL-3)METHYL]-5 TRIAZINE-1, 2, 4 ONE-6**

**EXEMPLE 44 : MORPHOLINOETHYL-1 PHENYL-3 [(FURYL-2)METHYL]-5 TRIAZINE-1,2,4 ONE-6**

**EXEMPLE 45 : MORPHOLINOETHYL-1 PHENYL-3 (NITRO-3 BENZYL)-5 TRIAZINE-1,2,4 ONE-6**

**EXEMPLE 46 : MORPHOLINOETHYL-1 PHENYL-3 (HYDROXY-3 BENZYL)-5 TRIAZINE-1,2,4 ONE-6**

**EXEMPLE 47 : MORPHOLINOETHYL-1 PHENYL-3 (METHYL-4 BENZYL)-5 TRIAZINE-1,2,4 ONE-6**

**EXEMPLE 48 : (MORPHOLINO-3 PROPYL)-1 PHENYL-3(CHLORO-3 BENZYL)-5 TRIAZINE-1,2,4 ONE-6**

En remplaçant dans l'exemple 36, stade D, le chlorhydrate de N-(chloro-2 éthyl)morpholine par le chlorhydrate de N-(chloro-3 propyl)morpholine, on obtient le produit de l'exemple.
Point de fusion : 164° C (oxalate acide)

**EXEMPLE 49 : PYRROLIDINOETHYL-1 PHENYL-3 (CHLORO-3 BENZYL)-5 TRIAZINE-1,2,4 ONE-6**

En remplaçant dans l'exemple 36, stade D, le chlorhydrate de N-(chloro-2 éthyl)morpholine par le chlorhydrate de N-(chloro-2 éthyl)pyrrolidine, on obtient le produit de l'exemple.
Point de fusion : 174° C (oxalate acide, monohydrate)

**EXEMPLE 50 : PYRROLIDINOETHYL-1 (CHLORO-3 PHENYL)-3 BENZYL-5 TRIAZINE-1,2,4 ONE-6**

En remplaçant dans l'exemple 29, stade D, le chlorhydrate de N-(chloro-2 éthyl) morpholine par le chlorhydrate de N-(chloro-2 éthyl)pyrrolidine, on obtient le composé de l'exemple.
Point de fusion : 188° C (oxalate acide)

**EXEMPLES 51 A 56**

En opérant comme dans l'exemple précedent mais en utilisant un chlorhydrate de chlorure d'aminoalkyle convenablement choisi, on obtient de la même façon

**EXEMPLE 51 : (MORPHOLINO-4 BUTYL)-1 (CHLORO-3 PHENYL)-3 BENZYL-5 TRIAZINE-1,2,4 ONE-6**

**EXEMPLE 52 : DIMETHYLAMINOETHYL-1 (CHLORO-3 PHENYL)-3 BENZYL-5 TRIAZINE-1,2,4 ONE-6**

**EXEMPLE 53 : N-METHYLPIPERAZINOETHYL-1 (CHLORO-3 PHENYL)-3 BENZYL-5 TRIAZINE-1,2,4 ONE-6**

**EXEMPLE 54 : N-PHENYLPIPERAZINOETHYL-1 (CHLORO-3 PHENYL)-3 BENZYL-5 TRIAZINE-1,2,4 ONE-6**

**EXEMPLE 55 : [(PERHYDROAZEPINYL-1) ETHYL]-1 (CHLORO-3 PHENYL)-3 BENZYL-5 TRIAZINE-1,2,4 ONE-6**

**EXEMPLE 56 : PIPERIDINOETHYL-1 (CHLORO-3 PHENYL)-3 BENZYL-5 TRIAZINE-1,2,4 ONE-6**

**EXEMPLE 57 : DIBENZYL-3,8 PHENYL-6 [1,2,4-TRIAZOLO][3,4-f][1,2,4]-TRIAZINE**

STADE A : CHLORO-6 BENZYL-5 PHENYL-3 TRIAZINE-1,2,4

Chauffer une solution de 15 mmoles (4 g) de phényl-3 benzyl-5 triazine-1,2,4 one-6 obtenue au stade C de l'exemple 28, dans 30 ml d'oxychlorure de phosphore à une température de 80° C. Après refroidissement, verser dans de l'eau glacée, alcaliniser et extraire à l'acétate d'éthyle. Sécher la phase organique, filtrer, évaporer à sec et purifier par chromatographie sur silice (éluant acétate d'éthyle/hexane : 25/75)

STADE B : DIBENZYL-3,8 PHENYL-6 [1,2,4-TRIAZOLO][3,4-f][1,2,4]-TRIAZINE

Chauffer pendant 12 heures une solution de 1,77 mmole du composé obtenu au stade précedent et de 1,5 équivalent (0,4 g) d'hydrazide de l'acide phénylacétique dans 10 ml de butanol. Après refroidissement, filtrer le précipité et le recristalliser dans l'éthanol.

Point de fusion : 165°C

**EXEMPLES 58 A 60**

En remplaçant dans l'exemple 57, stade B, l'hydrazide de l'acide phénylacétique par l'hydrazide de l'acide approprié, on obtient de la même façon les composés des exemples suivants :

**EXEMPLE 58 : DIPHENYL-3,6 BENZYL-8 [1,2,4-TRIAZOLO][3,4-f][1,2,4]-TRIAZINE**

Point de fusion : 190° C

**EXEMPLE 59 : METHYL-3 PHENYL-6 BENZYL-8 [1,2,4-TRIAZOLO] [3,4-f][1,2,4]-TRIAZINE**

Point de fusion : 181° C

**EXEMPLE 60 : PHENYL-6 BENZYL-8 [1,2,4-TRIAZOLO][3,4 -f][1,2,4]-TRIAZINE**

**EXEMPLE 61 : PHENYL-3 BENZYL-5 [(CHLORO-3 PHENYL)-4 PIPERAZINYL-1]-6 TRIAZINE-1,2,4**

Mettre en suspension 2 mmoles (570 mg) de chloro-6 benzyl-5 phényl-3 triazine-1,2,4 obtenue au stade A de l'exemple 57 dans 40 ml de butanol et ajouter 4 équivalents de (chloro-3 phényl)-1 pipérazine. Chauffer 12 heures à 120° C, concentrer la solution reprendre par de l'éther, filtrer le précipité obtenu et le recristalliser dans l'éthanol.

Point de fusion : 178° C

**EXEMPLE 62 : PHENYL-3 BENZYL-5 (METHYL-4 PIPERAZINYL-1)-6 TRIAZINE-1,2,4**

En procédant comme dans l'exemple 61 en utilisant la N-méthyl pipérazine, on obtient le produit de l'exemple.

**EXEMPLE 63 : PHENYL-3 BENZYL-5 DIETHYLAMINOETHYLAMINO-6 TRIAZINE-1,2,4**

En remplaçant dans l'exemple 61 la (chloro-3 phényl)-1 pipérazine par la diéthylaminoéthylamine, on obtient de la même façon le produit de l'exemple, salifié par un équivalent d'acide oxalique et recristallisé dans l'isopropanol.

Point de fusion : 168° C (oxalate acide)

**EXEMPLES 64 A 72 :**

En procédant comme dans l'exemple 63, en utilisant des chloro-6 triazine-1,2,4 convenablement substituées (intermédiaires de la préparation d'exemples précedemment décrits) qui sont condensées avec des amines appropriées, on obtient de la même façon les exemples suivants :

**EXEMPLE 64 : [(ETHYL-1 PYRROLIDINYL-2) METHYLAMINO]-6 BENZYL-5 PHENYL-3 TRIAZINE-1,2,4**

Point de fusion : 111° C

**EXEMPLE 65 : PIPERIDINOETHYLAMINO-6 BENZYL-5 PHENYL-3 TRIAZINE-1,2,4**

Point de fusion : 132° C

**EXEMPLE 66 : PIPERIDINOETHYLAMINO-6 BENZYL-5 (CHLORO-4 PHENYL)-3 TRIAZINE-1,2,4**

Point de fusion : 196° C (oxalate acide)

**EXEMPLE 67 : PIPERIDINOETHYLAMINO-6 METHYL-5 PHENYL-3 TRIAZINE-1,2,4**

Point de fusion : 124° C

**EXEMPLE 68 : PIPERIDINOETHYLAMINO-6 ISOPROPYL-5 (CHLORO-4 PHENYL)-3 TRIAZINE-1,2,4**

Point de fusion : 221° C (oxalate acide)

**EXEMPLE 69 : PIPERIDINOETHYLAMINO-6 METHYL-5 (CHLORO-4 PHENYL)-3 TRIAZINE-1,2,4**

Point de fusion : 215° C (oxalate acide)

**EXEMPLE 70 : PIPERIDINOETHYLAMINO-6 PHENYL-3 TRIAZINE-1,2,4**

Point de fusion : 105° C (monohydrate)

**EXEMPLE 71 : PIPERIDINOETHYLAMINO-6 PHENYL-3 (CHLORO-4 BENZYL)-5 TRIAZINE-1,2,4**

Point de fusion : 192° C (oxalate acide)

**EXEMPLE 72 : (PHENYL-3 TRIAZINE-1,2,4-YL-6)-1 [(FLUORO-4 BENZOYL)-3 PROPYL]-4 PIPERAZINE**

Point de fusion : 155° C (hémihydrate)

**EXEMPLE 73 : [(PIPERIDINO-3 PROPYL)AMINO]-6 (CHLORO-4 PHENYL)-3 BENZYL-5 TRIAZINE-1,2,4**

**EXEMPLE 74 : [(PIPERIDINO-4 BUTYL)AMINO]-6 (CHLORO-4 PHENYL)-3 BENZYL-5 TRIAZINE-1,2,4**

**EXEMPLE 75 : MORPHOLINOETHYLAMINO-6 BENZYL-5 PHENYL-3 TRIAZINE-1,2,4**

**EXEMPLE 76 : [(1,2,5,6-TETRAHYDROPYRID-1-YL)ETHYLAMINO]-6 METHYL-5 PHENYL-3 TRIAZINE-1,2,4**

**EXEMPLE 77 : [(-AZA-3 BICYCLO(3.3.0) OCT-3-YL)ETHYLAMINO]-6 BENZYL-5 PHENYL-3 TRIAZINE-1,2,4**

**EXEMPLE 78 : [(PERHYDROAZEPIN-1-YL)ETHYLAMINO]-6 BENZYL-5 PHENYL-3 TRIAZINE-1,2,4**

**EXEMPLE 79 : {[(PERHYDROAZEPIN-1-YL)-3 PROPYL]AMINO-}6 BENZYL-5 PHENYL-3 TRIAZINE-1,2,4**

**EXEMPLE 80 : {[(CHLORO-3 PHENYL)-4 PIPERAZIN-1-YL]ETHYLAMINO-}6 BENZYL-5 PHENYL-3 TRIAZINE-1,2,4**

**ETUDE PHARMACOLOGIQUE DES DERIVES DE L'INVENTION**

**EXEMPLE 81 : ETUDE DE L'ACTIVITE ANALGESIQUE**

L'activité sur la douleur a été recherchée chez la souris (20-25 g) selon un protocole dérivé de la technique décrite par KOSTER (GAIRIN et Coll, J. Pharmacol. Exp. Ther. (1988), **245**, 955). Les souris, réparties par randomisation en lots de 10 animaux, ont reçu le traitement par voie intrapéritonéale (excipient pour les témoins) 30 minutes avant l'injection intra-péritonéale d'une solution d'acide acétique à 1 %. Les étirements sont dénombrés entre la 5 ème et 20 ème minute après l'injection.

Le pourcentage d'activité obtenu a été évalué pour chaque dose (% de diminution du nombre d'étirements chez les traités par rapport aux témoins).

Il est apparu que les composés de l'invention possèdent une activité analgésique intéressante (activité 50 % proche pour certains composés de l'invention de 25 mg/Kg IP). De plus, aux doses testées, les produits sont totalement atoxiques.

**EXEMPLE 82 : ETUDE DE L'AFFINITE IN VITRO POUR LES RECEPTEURS DU SYSTEME NERVEUX CENTRAL**

Les tests d'affinité in vitro pour les récepteurs mu, delta, kappa, sigma, $5HT_{1A}$, $5HT_2$, $M_1$, $GABA_A$, $A_1$, $A_2$ ont été réalisés selon des techniques classiques de liaison aux récepteurs.

Les résultats de ces études montrent que les composés de l'invention possèdent des K0,5 de l'ordre de $10^{-6}$ M vis à vis des récepteurs sigma, M1, A1 et A2.

**EXEMPLE 83 : ACTIVITE INHIBITRICE DE L'ACETYL CHOLINESTERASE**

Le dosage de l'acétyl cholinestérase est effectué selon la méthode colorimétrique d'Ellman et Col. (Biochem. Pharmacol (1961), 7, 88-95). Sur ce test, les composés de l'invention montrent une très intéressante activité inhibitrice ($IC_{50}$ à $1.10^{-7}$ M).

**EXEMPLE 84 : COMPOSITION PHARMACEUTIQUE**

Comprimés dosés à 10 mg de morpholinoéthyl-1 phényl-3 isopropyl-5 triazine-1,2,4 one-6

FORMULE POUR 1000 COMPRIMES :

| | |
|---|---|
| Morpholinoéthyl-1 phényl-3 isopropyl-5 triazine-1,2,4 one-6 | 10 g |
| Amidon de blé | 15 g |
| Amidon de maïs | 15 g |
| Lactose | 65 g |
| Stéarate de magnésium | 2 g |
| Silice | 1 g |
| Hydroxypropyl cellulose | 2 g |

**Revendications**

1. Composés de formule générale I :

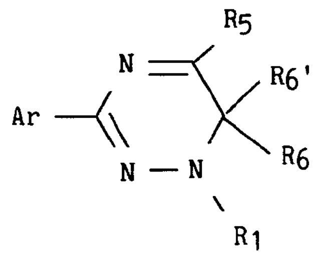

(I)

dans laquelle :

– Ar représente un radical aryle ou hétéroaryle, éventuellement substitué ,

– $R_5$ représente un atome d'hydrogène, un radical alkyle, cycloalkyle, cycloalkylalkyle, aryle, aralkyle, hétéroaryle, ou hétéroaralkyle éventuellement substitué sur le cycle aromatique ou hétéroaromatique, et

<u>soit</u> :

– $R_5$ et $R_6'$ représentent ensemble avec l'atome de carbone qui les porte une fonction C=O , et

– $R_1$ représente une chaîne

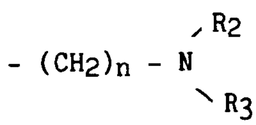

dans laquelle :

– n représente 2, 3, ou 4 ,

– $R_2$ et $R_3$, identiques ou différents représentent un atome d'hydrogène, un radical alkyle, ou forment ensemble, avec l'atome d'azote qui les porte, un hétérocycle choisi parmi morpholine, pyrrolidine, pipéridine, perhydroazépine, et pipérazine éventuellement substituée au niveau de l'autre atome d'azote par une chaîne alkyle ou un radical phényle éventuellement substitué, <u>soit</u> :

– $R_1$ et $R_6$ forment ensemble une liaison, et

– $R_5'$ représente un radical de formule :

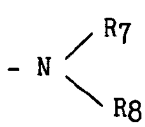

dans laquelle

. $R_7$ représente un atome d'hydrogène ou un radical alkyle,

. et $R_5$ représente un groupement de formule

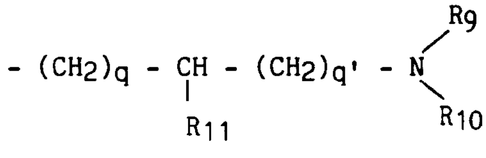

dans lequel

. q représente un nombre entier de 1 à 3,

. q' représente 0 ou 1,

. $R_9$, $R_{10}$, et $R_{11}$ représentent chacun indépendamment l'un de l'autre un atome d'hydrogène ou un alkyle,

. ou $R_9$ et $R_{10}$ forment ensemble avec l'atome d'azote qui les porte un hétérocycle de 5 à 10 sommets, mono ou bi-cyclique, saturé ou possédant une double liaison, incluant éventuellement dans son squelette un autre hétéroatome choisi parmi azote, oxygène, et soufre, étant entendu que lorsque $R_9$ et $R_{10}$ forment ensemble un hétérocycle contenant un second atome d'azote, cet

atome d'azote peut également être substitué par un radical alkyle, aryle ou hétéroaryle éventuellement substitué, ou par une chaîne de formule

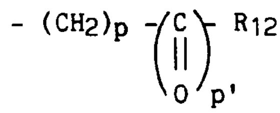

dans laquelle p représente 1, 2, ou 3, et p' représente 0 ou 1, $R_{12}$ représente un radical aryle ou hétéroaryle éventuellement substitué,

. ou $R_9$ et $R_{11}$ forment ensemble avec les atomes d'azote et de carbone qui les portent un hétérocycle de 5 à 7 sommets incluant éventuellement un autre hétéroatome dans son squelette un autre hétéroatome choisi parmi azote, oxygène, et soufre,

. ou $R_7$ et $R_8$ forment ensemble, avec l'atome d'azote qui les porte, une pipérazine substituée sur l'autre atome d'azote par un radical alkyle, par un radical phényle éventuellement substitué, ou par une chaîne de formule

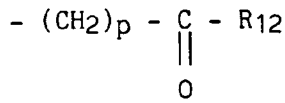

dans laquelle p et $R_{12}$ sont tels que définis précédemment,

soit :

$R_1$, $R_6$, et $R_6'$ forment avec le noyau triazinique qui les porte un système [1,2,4-triazolo][3,4-f][1,2-4]-triazine, le noyau triazolique étant substitué en position 3 par un groupement $R_{13}$ choisi parmi un radical alkyle, aryle, hétéroaryle, aralkyle, ou hétéroaralkyle éventuellement substitués sur le cycle aromatique ou hétéroaromatique,

leurs éventuels isomères optiques, isolés ou sous forme de mélange, ainsi que, le cas échéant, leurs sels d'addition à un acide pharmaceutiquement acceptable,

étant entendu que :

– le terme "substitué" signifie que les groupements qu'il affecte peuvent être substitués par un ou plusieurs radicaux, identiques ou différents, choisis parmi halogène, hydroxy, nitro, amino, trifluorométhyle, alkyl, et alkoxy, et/ou peuvent porter sur 2 atomes de carbone adjacents un groupement $-O-(CH_2)-O-$ dans lequel r représente un nombre entier de 1 à 3,

– sauf précision contraire, les termes "alkyle" et "alkoxy" signifient des groupements contenant de 1 à 6 atomes de carbone en chaîne droite ou ramifiée, et le terme "cycloalkyle" représente un groupement cyclique saturé comprenant de 3 à 7 atomes de carbone,

– le terme "aryle" représente un phényle ou un naphtyle et le terme "hétéroaryle" représente un groupement aromatique, mono ou bicyclique, comprenant de 5 à 10 sommets, et incluant dans son squelette carboné de 1 à 3 hétéroatomes choisis parmi azote, oxygène, et soufre.

**2.** Composés de formule I selon la revendication 1 répondant à la formule :

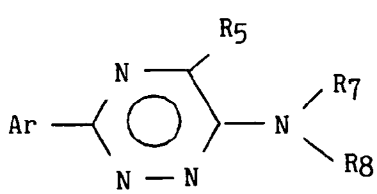

dans laquelle Ar, $R_5$, $R_7$, et $R_8$ ont les significations définies dans la revendication 1, leurs isomères optiques éventuels, isolés ou sous forme de mélange, ainsi que, le cas échéant, leurs sels d'addition à un acide pharmaceutiquement acceptable.

**3.** Composés de formule I selon les revendications 1 et 2 répondant à la formule :

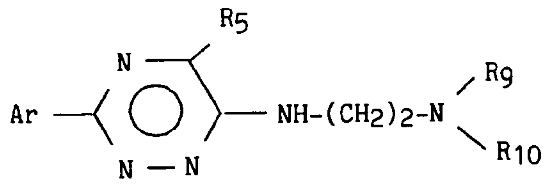

dans laquelle Ar, $R_5$, $R_9$, et $R_{10}$ ont les significations définies dans la revendication 1, leurs isomères optiques éventuels, isolés ou sous forme de mélange, ainsi que, le cas échéant, leurs sels d'addition à un acide pharmaceutiquement acceptable.

4. Composés de formule I selon la revendication 1 répondant à la formule :

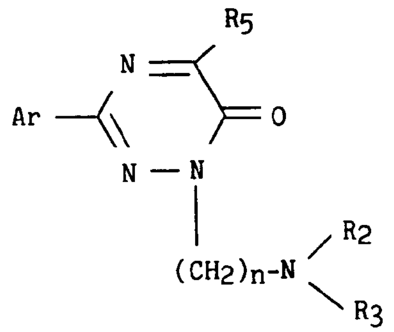

dans laquelle Ar, $R_2$, $R_3$, $R_5$, et n ont les significations définies dans la revendication 1, leurs isomères optiques éventuels, isolés ou sous forme de mélange, ainsi que, le cas échéant, leurs sels d'addition à un acide pharmaceutiquement acceptable.

5. Composés de formule I selon la revendication 1 répondant à la formule :

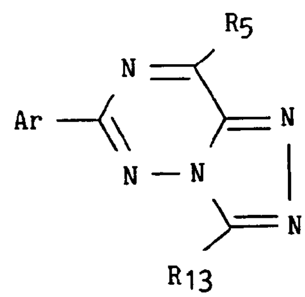

dans laquelle Ar, $R_5$, et $R_{13}$ ont les significations définies dans la revendication 1 leurs isomères optiques éventuels, isolés ou sous forme de mélange, ainsi que, le cas échéant, leurs sels d'addition à un acide pharmaceutiquement acceptable.

6. Composés de formule I selon l'une des revendications 1 à 5 dans lesquels $R_5$ représente un radical alkyle, aralkyle, hétéroaralkyle, ou un atome d'hydrogène, leurs isomères optiques éventuels, isolés ou sous forme de mélange, ainsi que, le cas échéant, leurs sels d'addition à un acide pharmaceutiquement acceptable.

7. Composé de formule I selon la revendication 1 qui est la pipéridinoéthylamino-6 benzyl-5 phényl-3 triazine-1,2,4, et ses sels d'addition à un acide pharmaceutiquement acceptable.

8. Composé de formule I selon la revendication 1 qui est la pipéridinoéthylamino-6 (chloro-4 benzyl)-5 phényl-3 triazine-1,2,4, et ses sels d'addition à un acide pharmaceutiquement acceptable.

9. Composés de formule I selon la revendication 1 qui est la pipéridinoéthylamino-6 isopropyl-5 (chloro-4

phényl)-3 triazine-1,2,4, et ses sels d'addition à un acide pharmaceutiquement acceptable.

**10.** Composés de formule I selon la revendication 1 qui est la méthyl-3 phényl-6 benzyl-8 [1,2,4-triazolo][3,4-f][1,2,4] triazine.

**11.** Procédé de préparation des composés de formule I selon la revendication 1, caractérisé en ce que l'on condense en milieu alcalin un α-aminoacide de formule II

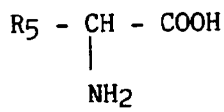

(II)

dans laquelle $R_5$ a la même signification que dans la formule I,
avec un halogénure d'acide de formule Ar - COX, dans laquelle Ar a la même signification que dans la formule I et X représente un atome d'halogène, pour obtenir un composé de formule III

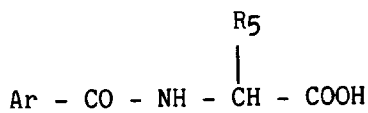

(III)

dans laquelle Ar et $R_5$ ont les significations ci-dessus définies, qui est ensuite :
a) **soit** estérifié en milieu acide par un alcool inférieur de formule R'-OH dans laquelle R' représente alkyle inférieur, pour conduire à un composé de formule IVa

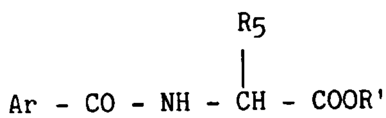

(IVa)

dans laquelle Ar, $R_5$, et R' ont les significations ci-dessus définies, qui est
– ou bien soumis en milieu anhydre à l'action d'un réactif de thionation suivie d'une acidification ou d'une extraction acidobasique pour obtenir un composé de formule Va

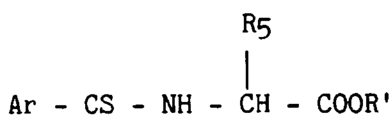

(Va)

dans laquelle Ar, $R_5$, et R' ont les significations ci-dessus définies,
– ou bien soumis à l'action d'un tétrafluoroborate de trialkyloxonium, dans un solvant anhydre pour obtenir un composé de formule Vb

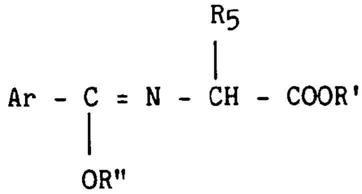

(Vb)

, dans laquelle Ar, $R_5$, et R' ont les significations ci-dessus définies, et R" représente un groupement alkyle,
les composés Va ou Vb étant ensuite cyclisés à chaud dans un solvant aprotique par action de l'hydrazine en un composé de formule VI

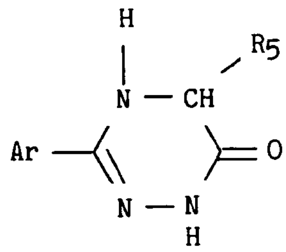

(VI)

dans laquelle Ar et $R_5$ ont les significations ci dessus définies, qui est ensuite deshydrogéné , soit par déhydrohalogénation, soit par action d'un réactif oxydant, en un composé de formule VII

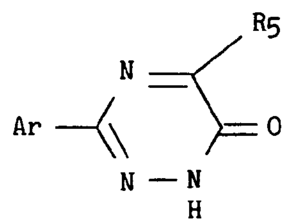

(VII)

dans laquelle Ar et $R_5$ ont la même signification que précédemment, qui est ensuite :

* soit traité successivement par un alcoolate de métal alcalin puis par un halogénure d'aminoalkyle de formule VIII

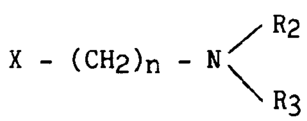

(VIII)

dans laquelle n, $R_2$, et $R_3$ ont la même signification que dans la formule I et X représente un atome d'halogène, pour obtenir un composé de formule Ia

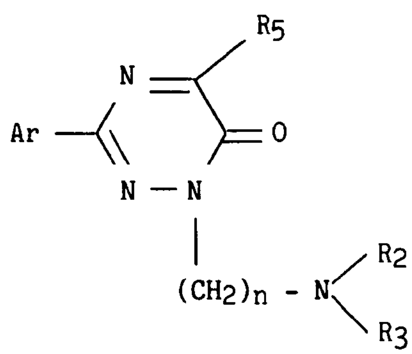

(Ia)

cas particulier des composés de formule I,

* soit soumis à l'action d'un composé polyhalogéné du phosphore, pour obtenir un composé de formule IX

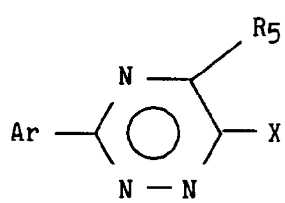

(IX)

dans laquelle Ar, $R_5$ et X ont les significations ci-dessus définies, qui est

– ou bien condensé avec une amine de formule X

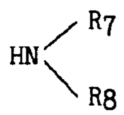

**(X)**

dans laquelle $R_7$ et $R_8$ ont la même signification que dans la formule I, pour obtenir un composé de formule Ib

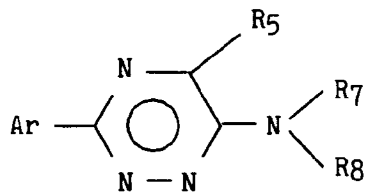

**(Ib)**

cas particulier des composés de formule I,

– ou bien condensé avec un hydrazide de formule

$$R_{13} - CO - NH - NH_2$$

dans laquelle $R_{13}$ a la même signification que dans la formule I, pour obtenir un composé de formule Ic

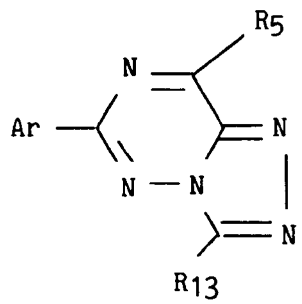

**(Ic)**

cas particulier des composés de formule I,

les composés Ia, Ib,et Ic qui forment l'ensemble des composés de formule I pouvant être si l'on désire salifiés par addition d'un acide pharmaceutiquement acceptable, et le cas échéant séparés en leurs différents isomères optiques,

b) **soit,** éventuellement, lorsque $R_5$ représente un atome d'hydrogène, condensé dans l'anhydride acétique en présence d'un acétate de métal alcalin, avec un aldéhyde aromatique de formule $Ar_1$ - CHO, dans laquelle $Ar_1$ représente un radical aryle ou hétéroaryle éventuellement substitué, pour obtenir un composé de formule Vc

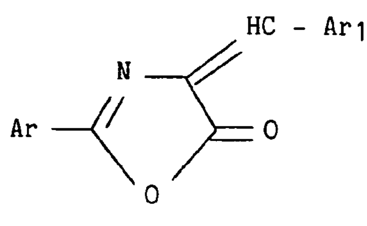

**(Vc)**

dans laquelle Ar et $Ar_1$ ont les significations ci dessus définies,

qui est soumis à l'action de l'hydrazine pour obtenir un composé de formule Vd

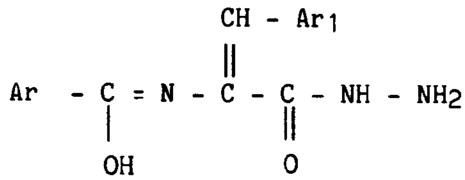

(Vd)

dans laquelle Ar et $Ar_1$ possèdent les significations ci dessus définies, qui chauffé en milieu alcalin conduit au composé de formule VIIa

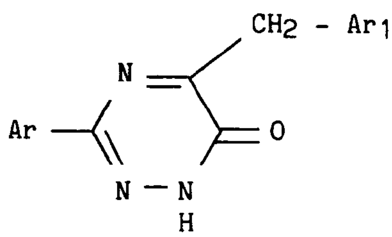

(VIIa)

cas particulier des composés de formule VII pour lesquels $R_5$ représente un radical arylméthyle ou hétéroaryl méthyle éventuellement substitué,
qui est soumis aux même opérations que les composés de formule générale VII pour obtenir respectivement les composés de formule Ia′, Ib′, et Ic′,

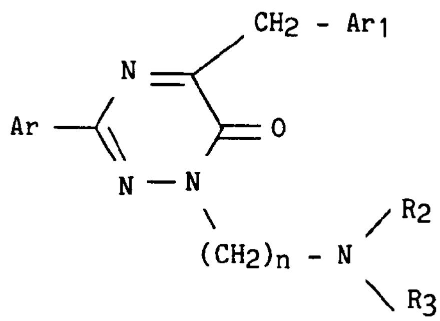

(Ia')

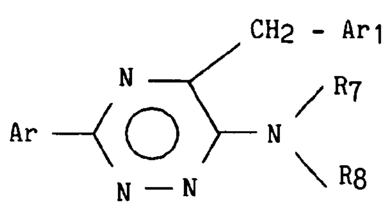

(Ib')

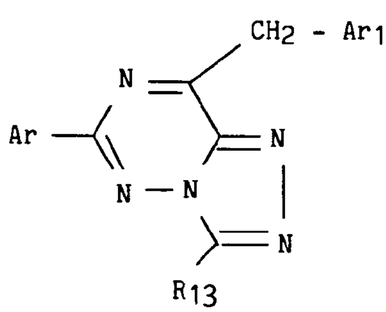

(Ic')

cas particuliers des composés de formule Ia, Ib, et Ic, qui sont si l'on désire salifiés par addition d'un acide pharmaceutiquement acceptable et le cas échéant séparés en leurs isomères optiques.

**12.** Composition pharmaceutique contenant comme principe actif au moins un composé selon la revendication 1 en combinaison avec un ou plusieurs excipients ou véhicules pharmaceutiquement acceptables.

**13.** Composition pharmaceutique selon la revendication 12 utilisable dans les troubles liés à un dysfonctionnement des récepteurs sigma, $A_1$, ou $A_2$, ou du système cholinergique.

**Revendications pour l'Etat contractant suivant : ES**

**1.** Procédé de préparation des composés de formule générale I :

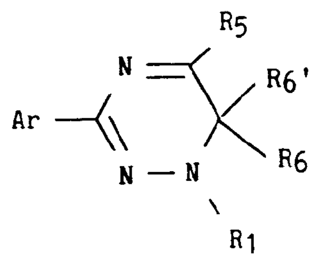

(I)

dans laquelle :

– Ar représente un radical aryle ou hétéroaryle, éventuellement substitué ,

– $R_5$ représente un atome d'hydrogène, un radical alkyle, cycloalkyle, cycloalkylalkyle, aryle, aralkyle, hétéroaryle, ou hétéroaralkyle éventuellement substitué sur le cycle aromatique ou hétéroaromatique, et

soit :

– $R_5$ et $R_6'$ représentent ensemble avec l'atome de carbone qui les porte une fonction C=O , et

– $R_1$ représente une chaîne

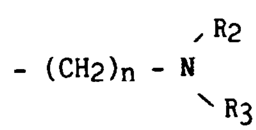

dans laquelle :

– n représente 2, 3, ou 4 ,

– $R_2$ et $R_3$, identiques ou différents représentent un atome d'hydrogène, un radical alkyle, ou forment ensemble, avec l'atome d'azote qui les porte, un hétérocycle choisi parmi morpholine, pyrrolidine, pipéridine, perhydroazépine, et pipérazine éventuellement substituée au niveau de l'autre atome d'azote par une chaîne alkyle ou un radical phényle éventuellement substitué, soit :

– $R_1$ et $R_6$ forment ensemble une liaison, et

– $R_5'$ représente un radical de formule :

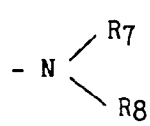

dans laquelle

. $R_7$ représente un atome d'hydrogène ou un radical alkyle,

. et $R_5$ représente un groupement de formule

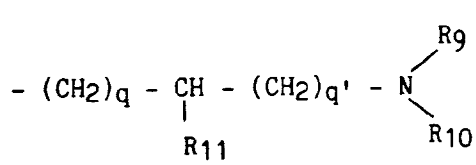

dans lequel

. q représente un nombre entier de 1 à 3,

. q′ représente 0 ou 1,

. $R_9$, $R_{10}$, et $R_{11}$ représentent chacun indépendamment l'un de l'autre un atome d'hydrogène ou un alkyle,

. ou $R_9$ et $R_{10}$ forment ensemble avec l'atome d'azote qui les porte un hétérocycle de 5 à 10 sommets, mono ou bi-cyclique, saturé ou possédant une double liaison, incluant éventuellement dans son squelette un autre hétéroatome choisi parmi azote, oxygène, et soufre, étant entendu que lorsque $R_9$ et $R_{10}$ forment ensemble un hétérocycle contenant un second atome d'azote, cet atome d'azote peut également être substitué par un radical alkyle, aryle ou hétéroaryle éventuellement substitué, ou par une chaîne de formule

$$-(CH_2)_p-\left(\underset{\underset{O}{\|}}{C}\right)_{p'}-R_{12}$$

dans laquelle p représente 1, 2, ou 3, et p′ représente 0 ou 1, $R_{12}$ représente un radical aryle ou hétéroaryle éventuellement substitué,

. ou $R_9$ et $R_{11}$ forment ensemble avec les atomes d'azote et de carbone qui les portent un hétérocycle de 5 à 7 sommets incluant éventuellement un autre hétéroatome dans son squelette un autre hétéroatome choisi parmi azote, oxygène, et soufre,

. ou $R_7$ et $R_8$ forment ensemble, avec l'atome d'azote qui les porte, une pipérazine substituée sur l'autre atome d'azote par un radical alkyle, par un radical phényle éventuellement substitué, ou par une chaîne de formule

$$-(CH_2)_p-\underset{\underset{O}{\|}}{C}-R_{12}$$

dans laquelle p et $R_{12}$ sont tels que définis précédemment, <u>soit</u> :

$R_1$, $R_6$, et $R_6'$ forment avec le noyau triazinique qui les porte un système [1,2,4-triazolo][3,4-f][1,2-4]-triazine, le noyau triazolique étant substitué en position 3 par un groupement $R_{13}$ choisi parmi un radical alkyle, aryle, hétéroaryle, aralkyle, ou hétéroaralkyle éventuellement substitués sur le cycle aromatique ou hétéroaromatique,

de leurs éventuels isomères optiques, isolés ou sous forme de mélange, ainsi que, le cas échéant, de leurs sels d'addition à un acide pharmaceutiquement acceptable,

étant entendu que :

– le terme "substitué" signifie que les groupements qu'il affecte peuvent être substitués par un ou plusieurs radicaux, identiques ou différents, choisis parmi halogène, hydroxy, nitro, amino, trifluorométhyle, alkyl, et alkoxy, et/ou peuvent porter sur 2 atomes de carbone adjacents un groupement $-O-(CH_2)-O-$ dans lequel r représente un nombre entier de 1 à 3,

– sauf précision contraire, les termes "alkyle" et "alkoxy" signifient des groupements contenant de 1 à 6 atomes de carbone en chaîne droite ou ramifiée, et le terme "cycloalkyle" représente un groupement cyclique saturé comprenant de 3 à 7 atomes de carbone,

– le terme "aryle" représente un phényle ou un naphtyle et le terme "hétéroaryle" représente un groupement aromatique, mono ou bicyclique, comprenant de 5 à 10 sommets, et incluant dans son squelette carboné de 1 à 3 hétéroatomes choisis parmi azote, oxygène, et soufre,

caractérisé en ce que :

on condense en milieu alcalin un α-aminoacide de formule II

$$R_5-\underset{\underset{NH_2}{|}}{CH}-COOH \qquad (II)$$

dans laquelle $R_5$ a la même signification que dans la formule I, avec un halogénure d'acide de formule Ar - COX, dans laquelle Ar a la même signification que dans la formule I et X représente un atome d'halogène, pour obtenir un composé de formule III

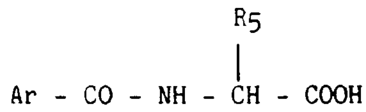

(III)

dans laquelle Ar et $R_5$ ont les significations ci-dessus définies, qui est ensuite :

a) soit estérifié en milieu acide par un alcool inférieur de formule R'-OH dans laquelle R' représente alkyle inférieur, pour conduire à un composé de formule IVa

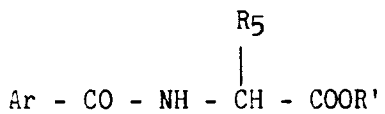

(IVa)

dans laquelle Ar, $R_5$, et R' ont les significations ci-dessus définies, qui est

– ou bien soumis en milieu anhydre à l'action d'un réactif de thionation suivie d'une acidification ou d'une extraction acido-basique pour obtenir un composé de formule Va

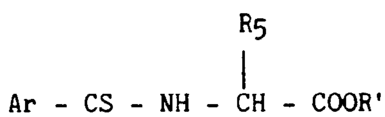

(Va)

dans laquelle Ar, $R_5$, et R' ont les significations ci-dessus définies,

– ou bien soumis à l'action d'un tétrafluoroborate de trialkyloxonium, dans un solvant anhydre pour obtenir un composé de formule Vb

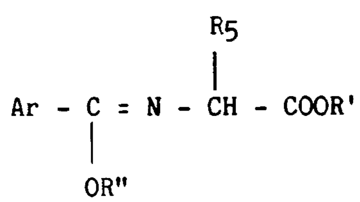

(Vb)

, dans laquelle Ar, $R_5$, et R' ont les significations ci-dessus définies, et R" représente un groupement alkyle,

les composés Va ou Vb étant ensuite cyclisés à chaud dans un solvant aprotique par action de l'hydrazine en un composé de formule VI

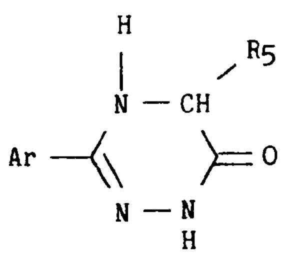

(VI)

dans laquelle Ar et $R_5$ ont les significations ci dessus définies, qui est ensuite deshydrogéné , soit par déhydrohalogénation, soit par action d'un réactif oxydant, en un composé de formule VII

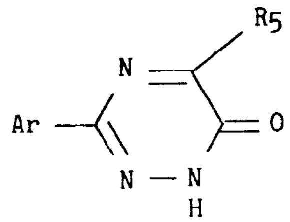

(VII)

dans laquelle Ar et $R_5$ ont la même signification que précédemment, qui est ensuite :

* soit traité successivement par un alcoolate de métal alcalin puis par un halogénure d'aminoalkyle de formule VIII

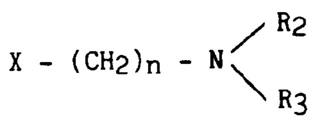

(VIII)

dans laquelle n, $R_2$, et $R_3$ ont la même signification que dans la formule I et X représente un atome d'halogène, pour obtenir un composé de formule Ia

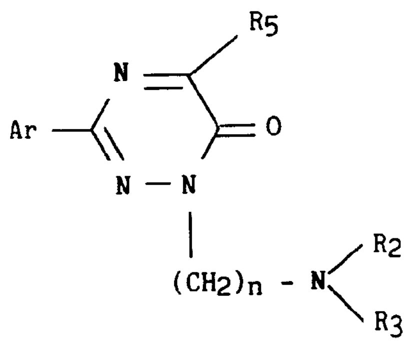

(Ia)

cas particulier des composés de formule I,

* soit soumis à l'action d'un composé polyhalogéné du phosphore, pour obtenir un composé de formule IX

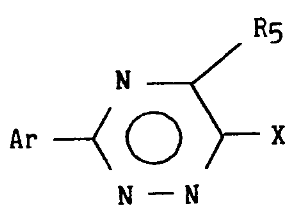

(IX)

dans laquelle Ar, $R_5$ et X ont les significations ci-dessus définies, qui est

– ou bien condensé avec une amine de formule X

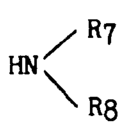

(X)

dans laquelle $R_7$ et $R_8$ ont la même signification que dans la formule I, pour obtenir un composé de formule Ib

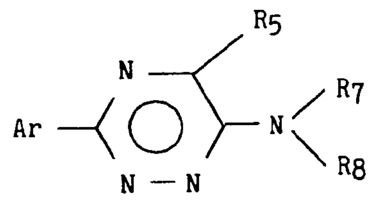

(Ib)

cas particulier des composés de formule I,

– ou bien condensé avec un hydrazide de formule

$$R_{13} - CO - NH - NH_2$$

dans laquelle $R_{13}$ a la même signification que dans la formule I, pour obtenir un composé de formule Ic

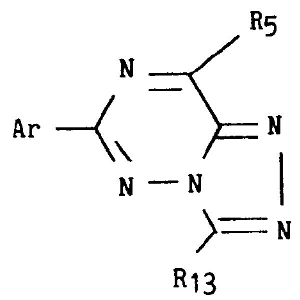

(Ic)

cas particulier des composés de formule I,

les composés Ia, Ib,et Ic qui forment l'ensemble des composés de formule I pouvant être si l'on désire salifiés par addition d'un acide pharmaceutiquement acceptable, et le cas échéant séparés en leurs différents isomères optiques,

b) **soit,** éventuellement, lorsque $R_5$ représente un atome d'hydrogène, condensé dans l'anhydride acétique en présence d'un acétate de métal alcalin, avec un aldéhyde aromatique de formule $Ar_1$ - CHO, dans laquelle $Ar_1$ représente un radical aryle ou hétéroaryle éventuellement substitué, pour obtenir un composé de formule Vc

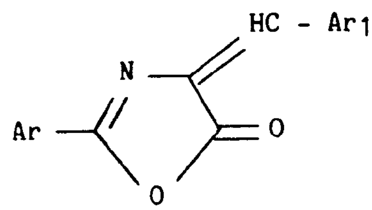

(Vc)

dans laquelle Ar et $Ar_1$ ont les significations ci dessus définies,

qui est soumis à l'action de l'hydrazine pour obtenir un composé de formule Vd

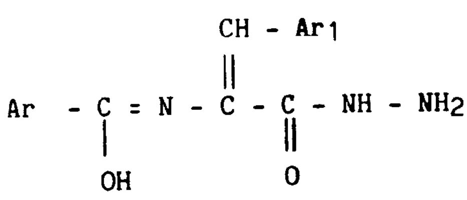

(Vd)

dans laquelle Ar et $Ar_1$ possèdent les significations ci dessus définies, qui chauffé en milieu alcalin conduit au composé de formule VIIa

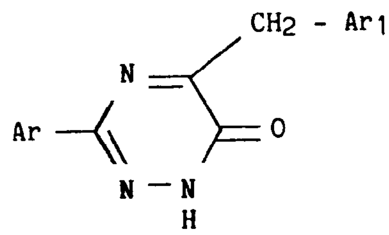

(VIIa)

cas particulier des composés de formule VII pour lesquels $R_5$ représente un radical arylméthyle ou hétéroaryl méthyle éventuellement substitué,
qui est soumis aux même opérations que les composés de formule générale VII pour obtenir respectivement les composés de formule Ia′, Ib′, et Ic′,

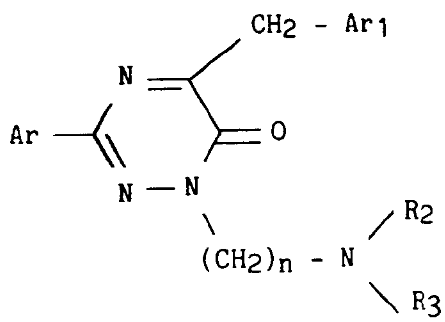

(Ia')

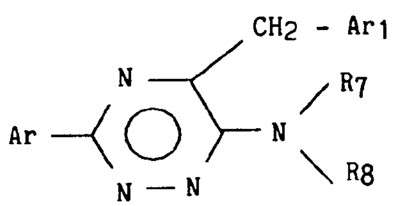

(Ib')

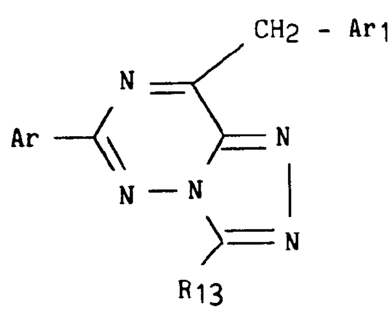

(Ic')

cas particuliers des composés de formule Ia, Ib, et Ic, qui sont si l'on désire salifiés par addition d'un acide pharmaceutiquement acceptable et le cas échéant séparés en leurs isomères optiques.

2. Procédé de préparation selon la revendication 1 des composés de formule I répondant à la formule :

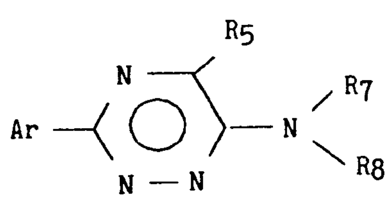

dans laquelle Ar, $R_5$, $R_7$, et $R_8$ ont les significations définies dans la revendication 1, de leurs isomères optiques éventuels, isolés ou sous forme de mélange, ainsi que, le cas échéant, de leurs sels d'addition à un acide pharmaceutiquement acceptable.

3. Procédé de préparation selon la revendication 1 des composés de formule I répondant à la formule :

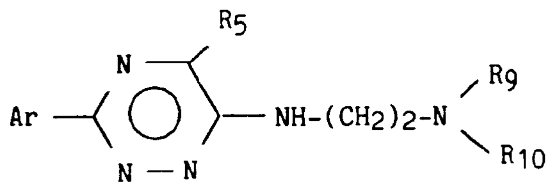

dans laquelle Ar, $R_5$, $R_9$, et $R_{10}$ ont les significations définies dans la revendication 1, leurs isomères optiques éventuels, isolés ou sous forme de mélange, ainsi que, le cas échéant, leurs sels d'addition à un acide pharmaceutiquement acceptable.

4. Procédé de préparation selon la revendication 1 des composés de formule I répondant à la formule :

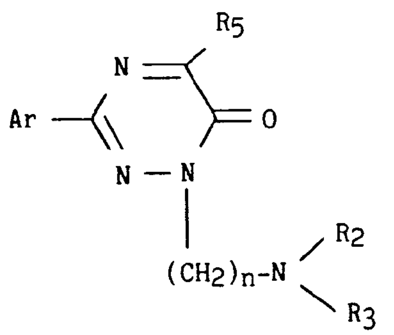

dans laquelle Ar, $R_2$, $R_3$, $R_5$, et n ont les significations définies dans la revendication 1, leurs isomères optiques éventuels, isolés ou sous forme de mélange, ainsi que, le cas échéant, leurs sels d'addition à un acide pharmaceutiquement acceptable.

5. Procédé de préparation selon la revendication 1 de composés de formule I répondant à la formule :

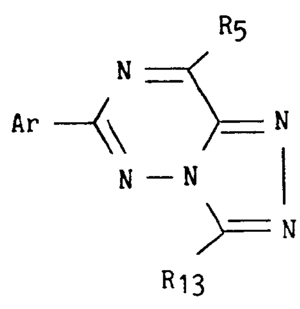

dans laquelle Ar, $R_5$, et $R_{13}$ ont les significations définies dans la revendication 1, de leurs isomères optiques éventuels, isolés ou sous forme de mélange, ainsi que, le cas échéant, de leurs sels d'addition à un acide pharmaceutiquement acceptable.

6. Procédé de préparation selon la revendication 1 des composés de formule I dans lesquels $R_5$ représente un radical alkyle, aralkyle, hétéroaralkyle, ou un atome d'hydrogène, de leurs isomères optiques éventuels, isolés ou sous forme de mélange, ainsi que, le cas échéant, de leurs sels d'addition à un acide pharmaceutiquement acceptable.

**7.** Procédé de préparation selon la revendication 1 de composé de formule I qui est la pipéridinoéthylamino-6 benzyl-5 phényl-3 triazine-1,2,4, et de ses sels d'addition à un acide pharmaceutiquement acceptable.

**8.** Procédé de préparation selon la revendication 1 du composé de formule I qui est la pipéridinoéthylamino-6 (chloro-4 benzyl)-5 phényl-3 triazine-1,2,4, et ses sels d'addition à un acide pharmaceutiquement acceptable.

**9.** Procédé de préparation selon la revendication 1 du composé de formule I selon la revendication 1 qui est la pipéridinoéthylamino-6 isopropyl-5 (chloro-4 phényl)-3 triazine-1,2,4, et de ses sels d'addition à un acide pharmaceutiquement acceptable.

**10.** Procédé de préparation selon la revendication 1 du composé de formule I selon la revendication 1 qui est la méthyl-3 phényl-6 benzyl-8 [1,2,4-triazolo][3,4-f][1,2,4] triazine.

**11.** Procédé de préparation d'une composition pharmaceutique contenant comme principe actif au moins un composé préparé selon l'une des revendications 1 à 10 en combinaison avec un ou plusieurs excipients ou véhicules pharmaceutiquement acceptables.

**12.** Procédé de préparation d'une composition pharamceutique selon la revendication 11 utilisable dans les troubles liés à un dysfonctionnement des récepteurs sigma, $A_1$, our $A_2$, ou des systèmes cholinergique.

**Revendications pour l'Etat contractant suivant : GR**

**1.** Procédé de préparation des composés de formule générale I :

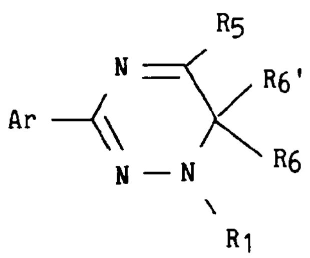

(I)

dans laquelle :

– Ar représente un radical aryle ou hétéroaryle, éventuellement substitué ,

– $R_5$ représente un atome d'hydrogène, un radical alkyle, cycloalkyle, cycloalkylalkyle, aryle, aralkyle, hétéroaryle, ou hétéroaralkyle éventuellement substitué sur le cycle aromatique ou hétéroaromatique, et

<u>soit</u> :

– $R_5$ et $R_6'$ représentent ensemble avec l'atome de carbone qui les porte une fonction C=O , et

– $R_1$ représente une chaîne

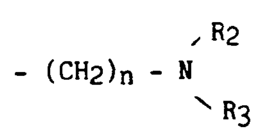

dans laquelle :

– n représente 2, 3, ou 4 ,

– $R_2$ et $R_3$, identiques ou différents représentent un atome d'hydrogène, un radical alkyle, ou forment ensemble, avec l'atome d'azote qui les porte, un hétérocycle choisi parmi morpholine, pyrrolidine, pipéridine, perhydroazépine, et pipérazine éventuellement substituée au niveau de l'autre atome d'azote par une chaîne alkyle ou un radical phényle éventuellement substitué, <u>soit</u> :

– $R_1$ et $R_6$ forment ensemble une liaison, et

– $R_5'$ représente un radical de formule :

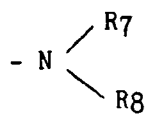

dans laquelle

. $R_7$ représente un atome d'hydrogène ou un radical alkyle,

. et $R_8$ représente un groupement de formule

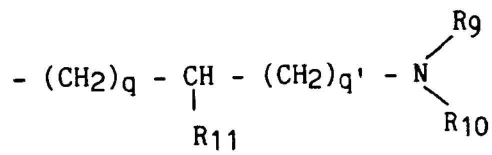

dans lequel

. q représente un nombre entier de 1 à 3,

. q' représente 0 ou 1,

. $R_9$, $R_{10}$, et $R_{11}$ représentent chacun indépendamment l'un de l'autre un atome d'hydrogène ou un alkyle,

. ou $R_9$ et $R_{10}$ forment ensemble avec l'atome d'azote qui les porte un hétérocycle de 5 à 10 sommets, mono ou bi-cyclique, saturé ou possédant une double liaison, incluant éventuellement dans son squelette un autre hétéroatome choisi parmi azote, oxygène, et soufre, étant entendu que lorsque $R_9$ et $R_{10}$ forment ensemble un hétérocycle contenant un second atome d'azote, cet atome d'azote peut également être substitué par un radical alkyle, aryle ou hétéroaryle éventuellement substitué, ou par une chaîne de formule

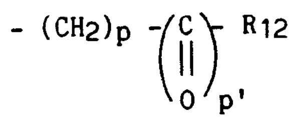

dans laquelle p représente 1, 2, ou 3, et p' représente 0 ou 1, $R_{12}$ représente un radical aryle ou hétéroaryle éventuellement substitué,

. ou $R_9$ et $R_{11}$ forment ensemble avec les atomes d'azote et de carbone qui les portent un hétérocycle de 5 à 7 sommets incluant éventuellement un autre hétéroatome dans son squelette un autre hétéroatome choisi parmi azote, oxygène, et soufre,

. ou $R_7$ et $R_8$ forment ensemble, avec l'atome d'azote qui les porte, une pipérazine substituée sur l'autre atome d'azote par un radical alkyle, par un radical phényle éventuellement substitué, ou par une chaîne de formule

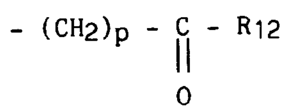

dans laquelle p et $R_{12}$ sont tels que définis précédemment,

soit :

$R_1$, $R_6$, et $R_6'$ forment avec le noyau triazinique qui les porte un système [1,2,4-triazolo][3,4-f][1,2-4]-triazine, le noyau triazolique étant substitué en position 3 par un groupement $R_{13}$ choisi parmi un radical alkyle, aryle, hétéroaryle, aralkyle, ou hétéroaralkyle éventuellement substitués sur le cycle aromatique ou hétéroaromatique,

de leurs éventuels isomères optiques, isolés ou sous forme de mélange, ainsi que, le cas échéant, de leurs sels d'addition à un acide pharmaceutiquement acceptable,

étant entendu que :

– le terme "substitué" signifie que les groupements qu'il affecte peuvent être substitués par un ou plusieurs radicaux, identiques ou différents, choisis parmi halogène, hydroxy, nitro, amino, trifluorométhyl-

thyle, alkyl, et alkoxy, et/ou peuvent porter sur 2 atomes de carbone adjacents un groupement -O-$(CH_2)$-O- dans lequel r représente un nombre entier de 1 à 3,

– sauf précision contraire, les termes "alkyle" et "alkoxy" signifient des groupements contenant de 1 à 6 atomes de carbone en chaîne droite ou ramifiée, et le terme "cycloalkyle" représente un groupement cyclique saturé comprenant de 3 à 7 atomes de carbone,

– le terme "aryle" représente un phényle ou un naphtyle et le terme "hétéroaryle" représente un groupement aromatique, mono ou bicyclique, comprenant de 5 à 10 sommets, et incluant dans son squelette carboné de 1 à 3 hétéroatomes choisis parmi azote, oxygène, et soufre,

caractérisé en ce que :

on condense en milieu alcalin un α-aminoacide de formule II

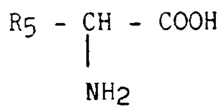

(II)

dans laquelle $R_5$ a la même signification que dans la formule I,

avec un halogénure d'acide de formule Ar - COX, dans laquelle Ar a la même signification que dans la formule I et X représente un atome d'halogène, pour obtenir un composé de formule III

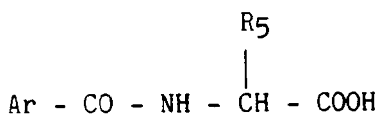

(III)

dans laquelle Ar et $R_5$ ont les significations ci-dessus définies,

qui est ensuite :

a) soit estérifié en milieu acide par un alcool inférieur de formule R'-OH dans laquelle R' représente alkyle inférieur, pour conduire à un composé de formule IVa

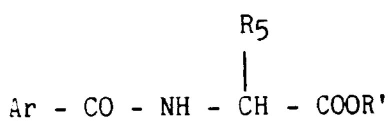

(IVa)

dans laquelle Ar, $R_5$, et R' ont les significations ci-dessus définies, qui est

– ou bien soumis en milieu anhydre à l'action d'un réactif de thionation suivie d'une acidification ou d'une extraction acido-basique pour obtenir un composé de formule Va

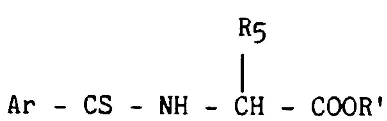

(Va)

dans laquelle Ar, $R_5$, et R' ont les significations ci-dessus définies,

– ou bien soumis à l'action d'un tétrafluoroborate de trialkyloxonium, dans un solvant anhydre pour obtenir un composé de formule Vb

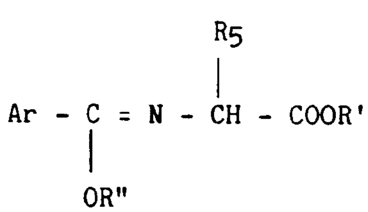

(Vb)

, dans laquelle Ar, $R_5$, et R' ont les significations ci-dessus définies, et R" représente un groupement alkyle,

les composés Va ou Vb étant ensuite cyclisés à chaud dans un solvant aprotique par action de l'hydrazine en un composé de formule VI

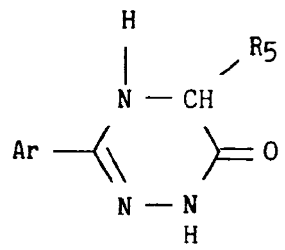

(VI)

dans laquelle Ar et $R_5$ ont les significations ci dessus définies, qui est ensuite deshydrogéné , soit par déhydrohalogénation, soit par action d'un réactif oxydant, en un composé de formule VII

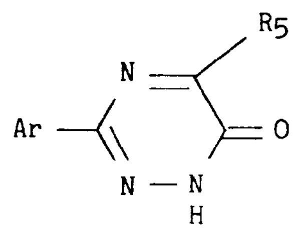

(VII)

dans laquelle Ar et $R_5$ ont la même signification que précédemment, qui est ensuite :

* soit traité successivement par un alcoolate de métal alcalin puis par un halogénure d'aminoalkyle de formule VIII

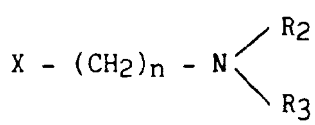

(VIII)

dans laquelle n, $R_2$, et $R_3$ ont la même signification que dans la formule I et X représente un atome d'halogène, pour obtenir un composé de formule Ia

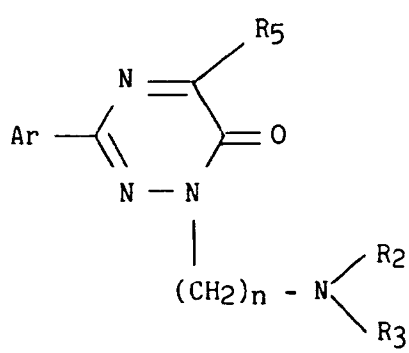

(Ia)

cas particulier des composés de formule I,

* soit soumis à l'action d'un composé polyhalogéné du phosphore, pour obtenir un composé de formule IX

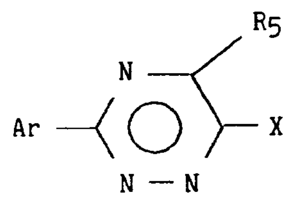

(IX)

dans laquelle Ar, $R_5$ et X ont les significations ci-dessus définies, qui est

– ou bien condensé avec une amine de formule X

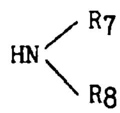

(X)

dans laquelle $R_7$ et $R_8$ ont la même signification que dans la formule I, pour obtenir un composé de formule Ib

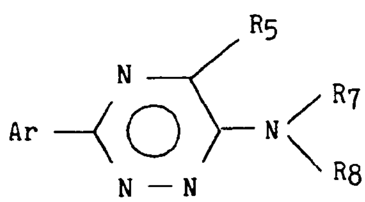

(Ib)

cas particulier des composés de formule I,

– ou bien condensé avec un hydrazide de formule

$$R_{13} - CO - NH - NH_2$$

dans laquelle $R_{13}$ a la même signification que dans la formule I, pour obtenir un composé de formule Ic

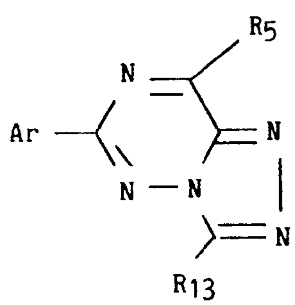

(Ic)

cas particulier des composés de formule I, les composés Ia, Ib,et Ic qui forment l'ensemble des composés de formule I pouvant être si l'on désire salifiés par addition d'un acide pharmaceutiquement acceptable, et le cas échéant séparés en leurs différents isomères optiques,

b) **soit,** éventuellement, lorsque $R_5$ représente un atome d'hydrogène, condensé dans l'anhydride acétique en présence d'un acétate de métal alcalin, avec un aldéhyde aromatique de formule $Ar_1$ - CHO, dans laquelle $Ar_1$ représente un radical aryle ou hétéroaryle éventuellement substitué, pour obtenir un composé de formule Vc

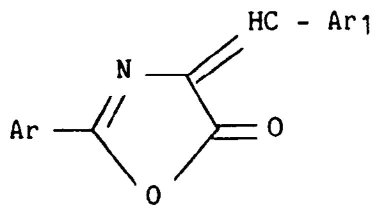

(Vc)

dans laquelle Ar et $Ar_1$ ont les significations ci dessus définies,
qui est soumis à l'action de l'hydrazine pour obtenir un composé de formule Vd

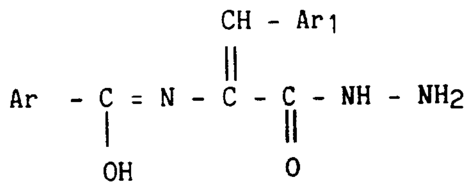

(Vd)

dans laquelle Ar et $Ar_1$ possèdent les significations ci dessus définies, qui chauffé en milieu alcalin conduit au composé de formule VIIa

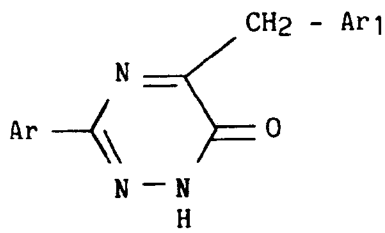

(VIIa)

cas particulier des composés de formule VII pour lesquels $R_5$ représente un radical arylméthyle ou hétéroaryl méthyle éventuellement substitué,
qui est soumis aux même opérations que les composés de formule générale VII pour obtenir respectivement les composés de formule Ia', Ib', et Ic',

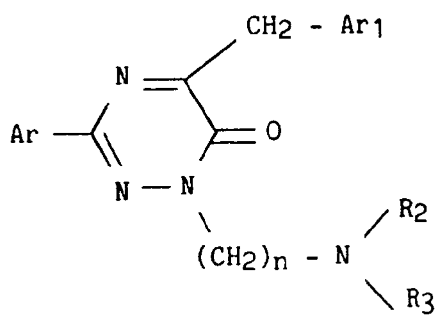

(Ia')

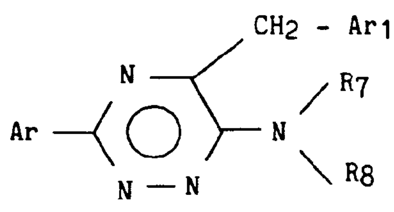

(Ib')

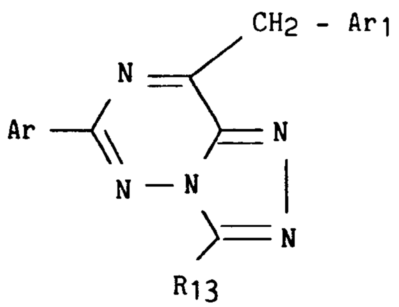

(Ic')

cas particuliers des composés de formule Ia, Ib, et Ic, qui sont si l'on désire salifiés par addition d'un acide pharmaceutiquement acceptable et le cas échéant séparés en leurs isomères optiques.

2. Procédé de préparation selon la revendication 1 des composés de formule I répondant à la formule :

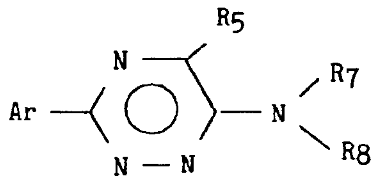

dans laquelle Ar, $R_5$, $R_7$, et $R_8$ ont les significations définies dans la revendication 1, de leurs isomères optiques éventuels, isolés ou sous forme de mélange, ainsi que, le cas échéant, de leurs sels d'addition à un acide pharmaceutiquement acceptable.

3. Procédé de préparation selon la revendication 1 des composés de formule I répondant à la formule :

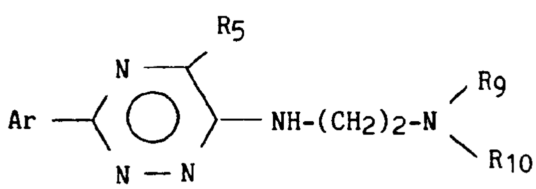

dans laquelle Ar, $R_5$, $R_9$, et $R_{10}$ ont les significations définies dans la revendication 1, leurs isomères optiques éventuels, isolés ou sous forme de mélange, ainsi que, le cas échéant, leurs sels d'addition à un acide pharmaceutiquement acceptable.

4. Procédé de préparation selon la revendication 1 des composés de formule I répondant à la formule :

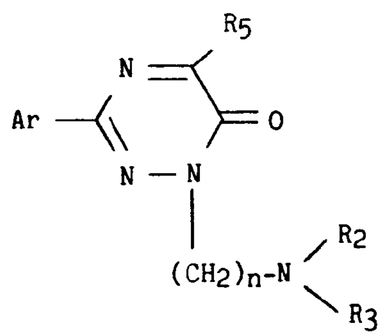

dans laquelle Ar, $R_2$, $R_3$, $R_5$, et n ont les significations définies dans la revendication 1, leurs isomères optiques éventuels, isolés ou sous forme de mélange, ainsi que, le cas échéant, leurs sels d'addition à un acide pharmaceutiquement acceptable.

5. Procédé de préparation selon la revendication 1 de composés de formule I répondant à la formule :

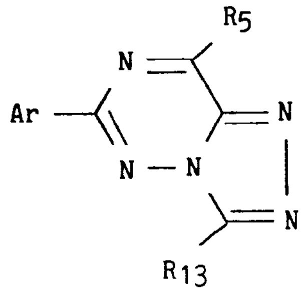

dans laquelle Ar, $R_5$, et $R_{13}$ ont les significations définies dans la revendication 1, de leurs isomères optiques éventuels, isolés ou sous forme de mélange, ainsi que, le cas échéant, de leurs sels d'addition à un acide pharmaceutiquement acceptable.

6. Procédé de préparation selon la revendication 1 des composés de formule I dans lesquels $R_5$ représente un radical alkyle, aralkyle, hétéroaralkyle, ou un atome d'hydrogène, de leurs isomères optiques éventuels, isolés ou sous forme de mélange, ainsi que, le cas échéant, de leurs sels d'addition à un acide pharmaceutiquement acceptable.

7. Procédé de préparation selon la revendication 1 de composé de formule I qui est la pipéridinoéthylamino-6 benzyl-5 phényl-3 triazine-1,2,4, et de ses sels d'addition à un acide pharmaceutiquement acceptable.

8. Procédé de préparation selon la revendication 1 du composé de formule I qui est la pipéridinoéthylamino-6 (chloro-4 benzyl)-5 phényl-3 triazine-1,2,4, et ses sels d'addition à un acide pharmaceutiquement acceptable.

9. Procédé de préparation selon la revendication 1 du composé de formule I selon la revendication 1 qui est la pipéridinoéthylamino-6 isopropyl-5 (chloro-4 phényl)-3 triazine-1,2,4, et de ses sels d'addition à un acide pharmaceutiquement acceptable.

10. Procédé de préparation selon la revendication 1 du composé de formule I selon la revendication 1 qui est la méthyl-3 phényl-6 benzyl-8 [1,2,4-triazolo][3,4-f][1,2,4] triazine.

11. Procédé de préparation d'une composition pharmaceutique contenant comme principe actif au moins un composé préparé selon l'une des revendications 1 à 10 en combinaison avec un ou plusieurs excipients ou véhicules pharmaceutiquement acceptables.

12. Procédé de préparation d'une composition pharamceutique selon la revendication 11 utilisable dans les troubles liés à un dysfonctionnement des récepteurs sigma, $A_1$, our $A_2$, ou des systèmes cholinergique.

Office européen des brevets

# RAPPORT DE RECHERCHE EUROPEENNE

Numero de la demande

EP 92 40 0095

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | EP-A-0 169 139 (SANOFI)<br>* page 16 - page 18; exemples 19,20 *<br>* page 22, ligne 29 - ligne 38 * | 1,2,6-9 | C07D253/06<br>A61K31/53<br>C07D403/12<br>C07D405/04<br>C07D487/04 |
| A | EP-A-0 099 438 (PIERRE FABRE S.A.)<br>* page 3 - page 4; exemples 1,2 *<br>* page 7; exemple 9 *<br>* page 13; revendication 4 * | 1,4,6 | |
| A | EP-A-0 080 296 (IMPERIAL CHEMICAL INDUSTRIES PLC )<br>* page 14 - page 15; exemple 3 *<br>* page 9, ligne 18 - ligne 20 *<br>* page 9, ligne 26 - ligne 28 * | 1,4,6 | |
| A | US-A-4 159 375 (R.I. TRUST ET AL.)<br>* colonne 8 - colonne 12; exemples 9,12-19 *<br>* colonne 3, ligne 63 - ligne 65 * | 1,5,6,10 | |
| | | | **DOMAINES TECHNIQUES RECHERCHES (Int. Cl.5 )** |
| | | | C07D |

Le présent rapport a été établi pour toutes les revendications

| Lieu de la recherche | Date d'achèvement de la recherche | Examinateur |
|---|---|---|
| LA HAYE | 31 MARS 1992 | FINK D.G. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)